# EUROPEAN PATENT SPECIFICATION

(11) **EP 1 520 585 B1**
(45) Date of publication and mention of the grant of the patent: **04.05.2011**
(21) Application number: 04022793.6
(22) Date of filing: 24.09.2004
(51) Int. Cl.: A61K 36/15, A61P 35/00

(54) **Cancer treatment using natural plant products or essential oils or components from some pistacia species**
Behandlung von Krebs mit natürlichen Pflanzenprodukten, etherischen Ölen oder Inhaltsstoffen von Pistazia Arten
Traitement du cancer avec des produits naturels de plantes, avec des huiles essentielles ou avec des composants à partir d'espèces de pistacia

(30) Priority: 02.10.2003 US 676101
(43) Date of publication of application: 06.04.2005
(73) Proprietor: Data Medica Padova S.p.A., 35133 Padova (IT)
(72) Inventor: Belloni Regazzo, Maria Paola, 35123 Padova (IT)
(74) Representative: Modiano, Micaela Nadia

(56) References cited:
- WO-A-03/092712
- GR-B- 1 003 868
- KARPOZILOS A ET AL: "The treatment of cancer in Greek antiquity" EUROPEAN JOURNAL OF CANCER, PERGAMON PRESS, OXFORD, GB, vol. 40, no. 14, September 2004 (2004-09), pages 2033-2040, XP004548627 ISSN: 0959-8049

## Description

The present invention relates, in general, to therapeutically effective natural products and pharmaceutical compositions containing plant essential oil from *Pistacia* species, or components, for the-prevention or the treatment of cancer in mammals, including humans, such as, for example, breast cancer.

It is known that nature represents a large source of therapeutically active drugs (Buffoni 1996). Indeed, the use of plants and other natural products for therapeutic purposes dates back basically to the beginning of humanity (Farnsworth 1985; Cragg. 1997).

Plants, like everything else, can be considered a mixture of different substances which despite a diversified metabolic fate, are maintained in a perfect equilibrium. Various drugs contain active principles with various pharmacologic activities, some of which are fundamental for some specific therapeutic use. Phytotherapy should not thus be considered an alternative cure, but rather an important sector of pharmacotherapy.

In fact, the phytotherapeutical remedies are often associate to the drugs of synthesis which, in various cases, are only to completion of therapies "natural".

The natural products represented for time an excellent source of medicinal for the care of the cancer. The anti-tumorals of natural origin which are used successfully in the clinical practice are therefore several and some of these also very well known such as, for example, taxol, isolated from *Taxus brevifolia*; vincristine and the vinblastine, isolated from *Vinca rosea*; etoposide and teniposide, semisynthetic-derivatives of podophyllotoxin, isolated from *Podophyllum peltatum.* Natural products, moreover, given their structural variety, continue to attract interest in the antitumoral field (Farnsworth, 1990; Cragg 1999).

Among plant natural products having potential pharmacological activity, essential oils, pure mixtures of organic substances, play a central role. Even in the same species the composition of an essential oil is very variable due to the plant high sensitivity to different climatic conditions. Essential oils are generally obtained by compression or hydro-distillation. The distillation in steam current is the most widely used extraction method. Given the very complex composition of essential oils and the many quantitative changes occurring during the vegetative cycle of the plant, their characterization is quite difficult.

The *Pistacia* genus (*Anacardaceae*) includes several species and is constituted by bushes or small trees, shrubs with resinous cortex.

The species found in the Mediterranean area are:
➢ *P. vera*
➢ *P. terebinthus*
➢ *P. lentiscus*

The oil obtained by the *Pistacia Vera* nuts is scarcely used and is present only in a limited number of pharmaceutical preparations, while, essential oil extracted from the resin of *Pistacia Terebinthus* has been shown to exert a significant anti-inflammatory activity in an experimental model of auricular inflammation in the rat (Giner-Larza 2000). Certainly much more used is instead the drug called rubber or mastic of *lentiscus* extracted by the *Pistacia Lentiscus.* This is used in pills as expectorant, while its tonic and astringent action is exploited for the care of children's diarrheas. Mastic is also chewed for its light antiseptic oral activity and sometimes it is associated to camphor, sandracca and peruvian balm to eliminate bad breath. The finding of the *lentiscus* resin in mummies dating back to the seventh century A.C. shows that the Egyptians used this substance to embalm the dead exploiting its antiseptic activity (Colombini 2000).

It is also known that:
➢ the water extract of *Pistacia Lentiscus,* rich of potassium, sodium and magnesium, induces in the rat a hypotensive activity (Sanz 1987, 1988), probably due to the presence of n-butanolic and ethyl in the extract;
➢ the essential oil obtained from the hydrodistillation of the resin of the resin of *lentiscus* exerts an antibacteric activity *in vitro* more marked toward the gram+, respect to gram- bacteria. Identifying, in the essential oil, a "natural"antibacteric action is of interest also because it could replace preserving substances, often suspected of toxicity, cancerogenity and theratogenity (Magiatis 1999);
➢ among the various extracts obtained by freeze-dried *P. lentiscus* leaves, the decoction is the only one to have *in vitro,* a good antibacteric activity in cultures of *Staphylococcus aureus*, *Sarcina lutea* and *Escherichia coli* and have a modest antimycotic activity, proven on cellular *Torulapsis glabrata* and *Candida Parapsilosis* cultures;
➢ the resin of the *lentiscus,* even when used at low dosages, acts quickly against peptic ulcer, thanks to its effectiveness against *Helicobacter Pylori* (Huwez 1998, Marone 2001).
➢ the cortex and leaves from *Pistacia lentiscus* are used against diarrhea and gonorrhea.

Essential oils from the *Pistacia* genus are rich in monoterpenes, which in fact represent the major components. Monoterpenes are non-nutritive dietary components also found in the essentials oils of many edible plants such as citrus, cherry, spearmint, dill, caraway, and others. Their natural functions may be as chemoattractants or chemorepellents, as they are largely responsible for the plant's pleasant fragrance. These simple 10 carbon isoprenoids are derived from the mevalonate pathway in plants but are not produced in mammals. For example, in spearmint and other plants, *d*-limonene is formed by the cyclization of geranylpyrophosphate by the enzyme limonene synthase (Croteau 1987). Limonene then serves as a precursor for other plant monocyclic monoterpenes such as carvone, carveol, and perillyl alcohol (Elson 1994).

The antitumor effects of dietary monoterpenes are attained with little or no host toxicity (Elson 1994, Crowell 1994 a, b, Evans 1995). A number of dietary monoterpenes have antitumor activity, exhibiting not only the ability to prevent the formation or progression of cancer, but to regress existing malignant tumors. Limonene and perillyl alcohol have well established chemopreventive activity against many cancer types. Indeed, *d-*limonene has a broad range of antitumor activities (Elson 1994, Crowell 1994). Dietary limonene reduces the incidence of spontaneous lymphomas in p53^{-/-} mice (Hursting 1995). Limonene, besides, has chemopreventive activity against spontaneous and chemically-induced rodent mammary, skin, liver, lung, and fore-stomach cancers, as well as *ras* oncogene-induced rat mammary cancer (Gould 1994). Furthermore, when administered either in pure form or as orange peel oil (95% *d*-limonene), limonene inhibits the development of chemically induced rodent mammary (Elegbede 1984, Elson 1988, Maltzman 1989, Wattenberg 1983), skin (Elegbede 1986 a), liver (Dietrich 1991), lung and forestomach (Wattenberg 1989, 1991) cancers (reviewed in Crowell and Gould 1994, Elson and Yu 1994, Elson 1995). In rat mammary carcinogenesis models, the chemopreventive effects of limonene are evident during the initiation phase of 7-12-dimethylbenz[a]anthracene (DMBA)² - induced cancer (Elson 1988) and during the promotion phase of both DMBA - and nitrosomethylurea (NMU) - induced cancers (Elson 1988, Maltzman 1989). Kawamori et al. (1996) reported that the development of azoxymethane-induced aberrant crypt foci in the colon of rats was significantly reduced when they were given 0.5% limonene in the drinking water. A Phase I clinical trial testing limonene's cancer chemotherapeutic activity is in progress (McNamee 1993).

Caraway seed oil, and its principal monoterpene, carvone, prevent chemically induced lung and forestomach carcinoma development when administered before the carcinogen (Wattenberg 1989). In addition, carveol (Crowell 1992) and menthol (Russin 1989) have chemopreventive activity against DMBA-induced rat mammary cancer when fed as 1% of the diet only during the initiation phase. Geraniol, an acyclic dietary monoterpene, has in vivo antitumor activity against murine leukemia, hepatoma and melanoma cells (Shoff 1991, Yu 1995) when administered before and after tumor cell transplantation.

In addition, many animal studies have shown perillyl alcohol to be a very powerful chemotherapeutics agent against several cancer types including pancreatic, breast, and liver cancer (Crowell 1999) and to have promotion phase chemopreventive activity against chemically induced liver cancer in rats (Mills 1995) and to be very effective at preventing tumor recurrences or secondary tumors in animals treated in a chemotherapy regimen (Haag 1994). Perillyl alcohol has chemotherapeutic activity against pancreatic cancer at doses that cause little toxicity to the host (Stark 1995). Perillyl alcohol reduced the growth of transplanted hamster pancreatic tumors to less than half that of controls. Moreover, a significant portion of perillyl alcohol-treated pancreatic tumors completely regressed, whereas none of the control tumors regressed (Stark 1995). Perillyl alcohol chemotherapy also reduces the growth rate of transplanted prostatic carcinomas in nude mice (Jeffers 1995). Thus, monoterpenes have chemotherapeutic activity against a number of solid types, including pancreatic cancer, one of the most refractory of all human cancers to available cancer therapies. The efficacy of perillyl alcohol chemotherapy against human cancer will be tested in forthcoming Phase I clinical trials (Phillips 1995)

Both limonene (Elegbede 1986 b, Haag 1992) and perillyl alcohol (Haag 1994) have chemotherapeutic activity against rat mammary tumors, causing the complete regression of > 80% of established DMBA- or NMU-induced rat mammary tumors with limonene and the aromatase inhibitor 4-hydroxyandrostenedione was more effective than either drug alone.

Several mechanisms of action may account for the chemotherapeutic activities of monoterpenes. The blocking chemopreventive effects of limonene and other monoterpenes during the initiation phase of mammary carcinogenesis are likely due to the induction of Phase II carcinogen-metabolizing enzymes, resulting in carcinogen detoxificatoin. The post-initiation phase, tumor suppressive chemopreventive activity of monoterpenes may be due in part to the inhibition of isoprenylation of cell-growth associated small G proteins such as p21 *ras* by limonene, perillyl alcohol, and their metabolites (Crowell 1991, 1994). This inhibition occurs at the level of the prenyl-protein transferases. In addition, perillyl alcohol affects the mevalonate pathway by inhibiting ubiquinone biosynthesis as well as the conversion of lathosterol to cholesterol (Ren 1994).

Chemotherapy of chemically-induced mammary tumors with monoterpenes results in tumor redifferentiation (Haag 1992). In limonene-treated mammary tumors, expression of the mannose-6-phosphate-insulin-like growth factor II receptor and transforming growth factor β1 are increased in the regressing, differentiating tumors, but not in the small number of tumors which are unresponsive to limonene (Jirtle 1993). In addition, the antitumor effects of dietary monoterpenes are attained with little or no host toxicity (Elson 1994, Crowell 1994, a,b, Evans 1995). In summary, a variety of dietary monoterpenes have been shown to be effective in the chemoprevention and chemotherapy of cancer. Now, monoterpenes research is progressing into human clinical trials for chemotherapeutic activity. Monoterpenes also possess many characteristics of ideal chemopreventive agents, namely, efficacious antitumor activity, commercial availability, low cost, oral bioavailability, low toxicity and novel mechanisms of action different from those of conventional cancer chemotherapeutic drugs, making it feasible to begin considering them for human cancer chemoprevention testing (Crowell 1996).

Several studies were led to the purpose to identify the chemical composition of the oil obtained by the leaves of *P. lentiscus.* Concluding that according to the geographic origin area the various oils are characterized by a monoterpene unusual, the myrcene is present in particular by 19-25% in the oil coming from Spain and Sicily (Calabro 1974, Boelens 1991) from the analysis of the studies taken back in literature; the α-pinene is present for 16% in those French (Buil 1975); the terpen-4-ol is present by 22% in the oil coming from Sardinia (Castola 2000) and δ-3-carene in the Egyptian oil 65% (De Pooter 1991). Present members in less amount are a few sesquiterpeni, what: D-germacrene (9%) (Boelens 1991), the β-caryophyllene (3.5-9%) (Buil 1975, Boelens 1991), δ-cadinene and α-cadinolo (6% of everybody) (Buil 1975), the β-bisabolene, β-bourbonene and caryophyllene oxide (about 3-4% of everybody) (De Pooter 1991). The concentrations of the monoterpenes, besides, significantly change if the oil is obtained by the fruit. In particular, comparing two oils, the one coming from Spain (Boelens 1991) and the one from Australia (Wyllie 1990), it obtains, what component majors, respectively myrcene (72 and 39%), α-pinene (10 and 28%) and the limonene (87 and 11%).

The oils obtained for hydrodistillation by the mastic coming from Spain and Greece are instead characterized by a high α-pinene content (65-86%) and a low myrcene (3-25%) content (Scurbis 1975, Papageorgiou 1981; Katsiotis 1984; (Boelens 1991).

Of particular interest is the work of Migiatis and coll. (1999) who, using gas-cromatography and mass spectroscopy, identified 69 members of essential treols of *P. lentiscus*, var. chia, respectively obtained from the leaves, the twigs and the mastic.

GR 1 003 868 discloses the use of a product deriving from the plant Pistacia lentiscus var. chia of the Anacardiaceae family as an antioxidant, as wound healing inductor and as cytostatic.

WO 03/092712, that is cited as prior art under Article 54(3) EPC, discloses the citostatic or cytotoxic activity of the Pistacia lentiscus var. chia.

Karpozilos A et al., "The treatment of cancer in Greek antiquity", European Journal of Cancer, Pergamon Press, Oxford, GB, vol. 40, no. 14, September 2004, pages 2033-2040, that is cited as prior art under Article 54(3) EPC, that discloses that Archigenes of Apameia, Galen of Pergamon and Oreibasios of Pergamon reported on the use of P. terebinthus in the treatment of cancer.

The present invention deals with the use of *Pistacia* natural products or essential oils and/or components, natural or synthetic, or mixtures or derivatives, and possibly other related natural products thereof for cancer prevention and treatment. In particular the present invention relates to the use of the above mentioned, by either oral or parenteral administration, also as adjuvant in combination with other cures, in preventive and therapeutic regimens directed towards the inhibition of cell growth or the killing of tumoral cells in humans and other animal species.

Additional objects and attendant advantages of the present invention will be set forth, in part, in the description that follows, or may be learned from practicing or using the present invention.

The objects and advantages may be realized and attained by means of the features and combinations particularly recited in the appended claims.

It is to be understood that the foregoing general description and the following detailed description provide the experimental basis for the invention, are exemplary and explanatory only and are not to be viewed as being restrictive of the invention, as claimed.
Figure 1 shows the cytotoxic effect of Lentiscus oil from Portugal.
Figure 2a, 2b ancd 2c show the cytotoxic effect of single oil components.
Figure 3 shows the cytotoxic effect of DM1C on MCF-7 cells.
Figure 4 shows the cytotoxic effect of DM2A1 on 2008 and LoVo cells.
Figure 5 shows the cytotoxic effect of DM3Z on MCF-7, 2008 and LoVo cells.
Figure 6 shows the cytotoxic effect of DMF1 on MCF-7, 2008 and LoVo cells.
Figure 7 shows the cytotoxic effect of DMP on 2008 cells.
Figure 8 shows the cytotoxic effect of DMG2 on LoVo cells.
Figure 9 shows the cytotoxic effect of DM3P on LoVo cells.
Figure 10 shows the cytotoxic effect of DM72 on MCF-7, 2008 and LoVo cells.
Figure 11 shows the cytotoxic effect of DM2Z on 2008 cells.
Figure 12 shows the cytotoxic effect of DM1Z on 2008 and LoVo cells.
Figure 13 shows the cytotoxic effect of DMF3 on MCF-7, 2008 and LoVo cells.
Figure 14 shows the cytotoxic effect of DMV2X on LoVo cells.
Figure 15 shows the cytotoxic effect of DMV5X on MCF-7, 2008 and LoVo cells.
Figure 16 shows the cytotoxic effect of DM4a on LoVo cells.
Figure 17 shows the cytotoxic effect of DM4p on 2008 and LoVo cells.
Figure 18 shows the cytotoxic effect of DM5a on 2008 cells.
Figure 19 shows the cytotoxic effect of DM4C on MCF-7, 2008 and LoVo cells.
Figure 20 shows the cytotoxic effect of DMK on 2008 cells.
Figure 21 shows the cytotoxic effect of DMF2 on MCF-7, 2008 and LoVo cells.
Figure 22 shows the cytotoxic effect of DM1P on MCF-7 cells.
Figure 23 shows the cytotoxic effect of DMG1 on MCF-7 and 2008 cells.
Figure 24 shows the cytotoxic effect of DM2C on LoVo cells.
Figure 25 shows the cytotoxic effect of DM3C on LoVo cells.
Figure 26 shows the cytotoxic effect of DM2P on LoVo cells.
Figure 27 shows the cytotoxic effect of DM1S on MCF-7 cells.
Figure 28 shows the cytotoxic effect of DMNP on 2008 and LoVo cells.
Figure 29 shows results of cytofluorimetric analysis.
Figure 30 shows an evaluation of the nitrite concentration.

The present invention regards the use for manufacturing a medicament for treating or preventing cancer in a mammal, including a human, comprising administrating an effective amount of an essential oil obtained from a plant of *Pistacia* genus selected from P. vera and P. Integerrima.

The present invention also regards the use of bornyl acetate for manufacturing a medicament for treating colon and ovary adenocarcinoma.

"Products obtained from a plant of the *Pistacia* genus" as used in the present specification and claims, means any part of a plant of *Pistacia* genus, as leaves, twigs, seeds, routs, nuts, galls, berries, branches, flowers, any natural product of a plant of *Pistacia* genus, as resins, products obtained from a plant of the *Pistacia* genus by any technique, for example but not limited to extraction, grinding, chemical, physical or chemical-physical treatments.

"Plant of *Pistacia* genus" as used in the present specification and claims means

one of the species *P. vera* and *P. irrtegerrima.* In one embodiment of the present invention, the plant of *Pistacia* genus is of European and Asiatic origin.

In particular the present invention relates to the use of the above mentioned essential oils, by either oral or parenteral administration, also as adjuvant in combination with other cures, in preventive and therapeutic regimens directed towards the inhibition of cell growth or the killing of tumoral cells in humans and other animal species.

The features and advantages of the present invention will become more clearly appreciated from the following description of experimental data indicating the *in vitro* anti-tumoral activity of essential oils extracted from various species of *Pistacia*.

### METHODS:

### Plant Collection:

Aerial parts (leaves and twigs, branches, fruits, nuts, seeds, flowers and galls) of the plants were collected in different seasons and at various times of the day in three different Italian regions: Veneto (*P. terebinthus*); Tuscany (*P. lentiscus*); Sicily (*P*.*vera*). Other samples were collected outside Italy: one of the *P. lentiscus* samples was collected in Portugal, while the *P. integerrima* plants were collected in Nepal.

Plant parts in most cases were collected and then rinsed, dried and frozen at -21°C within three hous from collection. The material was then hydro-distilled within few months. The vegetable material exposed to hydro-distillation consisted in leaves, flowers, fruits, branches, galls and nuts of *P. terebinthus, P. lentiscus, P. vera* and/or *P*.*integerrima*. Samples were washed and dried carefully and preserved to low temperature (-21°) to keep unchanged their phytochemical composition until the moment of the distillation. All the samples, before being exposed to distillation, were minced to obtain the maximum extraction yield and make the process of diffusion of the essence easier (Boelens 1991, De Pooter 1991, Magiatis 1999, Papageorgiou 1981).

### Essential Oil Extraction

The equipment used for the extraction of the essential oils consisted in a container in Inox steel (101t), in which 1750 ml of distilled water are added, separated from the minced drug by a steel grid, to avoid direct contact of the drug with the extraction water. The sample is compacted by a further grill to avoid handling of the drug during the extraction proceeding and at the same time let the water steam freely flow down. To this point the steel container closed. The boiler is equipped with a thermometer, to be able to check at every moment of the distillation the temperature in boiler, and is also connected to a distillation column in steel, taking a coolant to the superior extremity, always in steel, with cooling running water. Water in the range container to the ebullition develops steam which going beyond the grill, laps the drug and extracts the essences contained, gone beyond the grill, the steam is directed along the distillation column and condensed in the coolant; the water mixture-essences to this point is collected in a graded cylinder containing some ethilic ether to dissolve the essential oil extracted by the steam, which for their liophylic nature, present a greater affinity for the solvent. The organic phase is then treated with natrium sulphate anhidrous, filtered and evaporated (Boelens 1991, De Pooter 1991, Magiatis 1999, Papageorgiou 1981). The distillation was usually led for up to 4 hours with constant heating; the mean initial weight of the sample was: leaves (450 g), branches (250 g), berries (100 g), flowers ( 270 g ). The yield for the various parts of the plant, express as percent of initial wet weight was: leaves (0,05%), branches (0,06%), berries (0,11%), flowers (0,08%), galls (0,40%).

### Determination of oil chemical composition

The chemical composition of the essential oils obtained by P. *lentiscus, P. terebinthus, P. Vera* and *P. integerrima* was determined by means of analysis gascromatographic coupled to a detector mass spectrophotometer (GC/MS), using an operating system Hewlett-Packard 6890-5973 in endowed modality EI (electronic ionization with potential 70 eV), equipped of capillary columne HP-5 MS (30 m x 0.25 mm), with thickness of the equal film to 0.25 m, stationary phase of polidimetil silossano al 95%. It was operated applying one program of temperature starting from 60°C for the first three minutes raising up to 280 ° with a speed of 3°C/min. for 5 minutes; the Injector was kept to 200 °C. The spectrum obtained was compared with Wiley library's mass spectra (Boelens 1991, De Pooter 1991, Magiatis 1999, Papageorgiou 1981).

### Biological Assays

Samples preparation: the stock solution of essential oils (9%) were prepared in DMSO (1%) and in culture medium (90%). All the procedures were carried out under sterile conditions. Before each experiment the stock solutions were diluted with growth medium and used immediately.

Cell Lines: Cytotoxicity was evaluated on three human adenocarcinoma cell lines: ovarian (2008), breast (MCF-7), colon (LoVo).

The human ovarian adenocarcinoma cell line 2008, kindly supplied by Prof. G. Marverti (Department of Biomedical Sciences, University of Modena) and were maintained in RPMI 1640 medium supplemented with 10% heat-inactivated FCS (Foetal Calf Serum), 1% antibiotics (all products of Biochrom KG Seromed, Berlin), and 1% 2 mM glutamine (Merck).

The human breast adenocarcinoma cells lines MCF-7, supplied by the Experimental Zooprophylaxis Institute of Lombardy and Emilia (Brescia, Italy), were cultured in MEM with Eagle's salts, plus 10% heat-inactivated Foetal Calf Serum, 1% antibiotics and sodium piruvate (all products of Biochrom KG Seromed), 1% 200 mM glutamine (Merck).

The human colon adenocarcinoma cell line LoVo, kindly supplied by Dr. G. Toffoli, Oncologic Reference Centre, Aviano, Italy. Tle cell line was cultured in Ham's F12 with the Addition of 10% heat inactivated Foetal Calf Serum, 1% glutamine 200 mM (Merk), 1% natrium piruvate (Seromed Biochrom KG, Berlin).

Cytotoxicity: The cells (1x10⁵ cells/ml) were seeded in 96-well tissue plates (Falcon) and treated 24 h later with each essential oil at different concentrations. After 3 h exposure, medium was discarded, the plates were washed with sterile PBS and then added with growth medium.

The cytotoxic effect was evaluated was by tetrazolium salts reduction assay (MTT) after 21h of incubation. An amount of 20 µL of MTT solution (5 mg/mL in PBS) was added to each well, and plates were incubated for 4 h at 37°C. DMSO (150 µL) was added to all wells and mixed thoroughly to dissolve the dark-blue crystals. The absorbance was measured on a microculture plate reader (Titertek Multiscan) using a test wavelength of 570 nm and a reference wavelength of 630 nm.

*Statistical analysis*: The IC₅₀ was determined by linear regression analysis, after logit transformation (Rodbard 1975) and the dose-response curve best-fit was elaborated by Software "GraphPad Prism Version 3.0". For each assay the experiments were performed in triplicate and each essential oil was tested in triplicate on three different cell lines.

Nitrite assay: The nitrite concentration in the culture medium was measured as an indicator of NO production using Griess reaction. One hundred microliters of each supernatant was mixed with the same volume of Griess A reagent (1% sulphanilamide in 5% phosphoric acid) and after 10 minutes 100 ml of Griess B reagent (0.1% naphthylethylenediamine dihydrochloride in water) was added. After 15 minutes the absorbance of mixture was determined at 543 nm.

Cytofluorimetry: cells are collected and rinsed twice with cold PBS and then resuspended in 1X binding buffer at a concentration of 1 x 106 cells/ml. Transfer 100 µl of the solution (1 x 105 cells) to a 5 ml culture tube. 5 µl of Annexin V-FITC and 5µl of PI are added. Cells are gently vortexed and incubated for 15 min at RT (25°C) in the dark. 400 µl of 1X binding buffer to each tube are then added and samples analyzed by flow cytometry within one hour. Annexin V is a 35-36 kDa Ca2+ dependent phospholipid-binding protein that has a high affinity for PS, and binds to cells with exposed PS. Annexin V may be conjugated to fluorochromes such as Propidium Iodide (PI). This format retains its high affinity for PS and thus serves as a sensitive probe for flow cytometric analysis of cells that are undergoing apoptosis. Since externalization of PS occurs in the earlier stages of apoptosis, Annexin V-FITC staining can identify apoptosis at an earlier stage than assays based on nuclear changes such as DNA fragmentation. Annexin V-FITC staining precedes the loss of membrane integrity which accompanies the latest stages of cell death resulting from either apoptotic or necrotic processes. Therefore, staining with Annexin V-FITC is typically used in conjunction with a vital dye such as Propidium Iodide to allow the investigator to identify early apoptotic cells (Annexin V-FITC positive, PI negative). For example, cells that are viable are Annexin V-FITC and PI negative; cells that are in early apoptosis are Annexin V-FITC positive and PI negative; and cells that are in late apoptosis or already dead are are both Annexin V-FITC and PI positive. This assay does not distinguish, per se, between cells that have already undergone apoptotic death and those that have died as a result of a necrotic pathway because in either case, the dead cells will stain with both Annexin-FITC and PI. However, when apoptosis is measured over time, cells can be often tracked from Annexin V-FITC and PI negative (viable, or no measurable apoptosis), to Annexin V-FITC positive and PI negative (early apoptosis, membrane integrity is present) and finally to Annexin V-FITC and PI positive (end stage apoptosis and death). The movement of cells through these three stages suggests apoptosis. In contrast, a single observation indicating that cells are both Annexin V-FITC and PI positive, in of itself, reveals less information about the process by which the cells underwent their demise.

### Reagents

1. Annexin V-FITC
2. Propidium Iodide.
3. 10X Annexin V Binding Buffer.

### Staining

1. Wash cells twice with cold PBS and then resuspend cells in 1X binding buffer at a concentration of 1 x 106 cells/ml.
2. Transfer 100 µl of the solution (1 x 105 cells) to a 5 ml culture tube.
3. Add 5 µl of Annexin V-FITC and 5 µl of PI.
4. Gently vortex the cells and incubate for 15 min at RT (25°C) in the dark.
5. Add 400 µl of 1X binding buffer to each tube. Analyze by flow cytometry within one hour.

The results are shown in Table 1a,Table 1b and Table 1c.

The results shown in Table 1a and Table 1b do not regard the claimed invention.

**Table la- P. lentiscus Composition**

| **RT*** | **Oil Composition** | | **Leaves** | | | | | **Fruits** | | **Branches** | |
|---|---|---|---|---|---|---|---|---|---|---|---|
| | | **Portuguese Oil** | **DM1C** | **DM2A1** | **DM3Z** | **DMF1** | **DMP** | **DMG2** | **DM3P** | **DM72** | **DM2Z** |
| 6.08 | Tricyclene | - | 0.33 | 0.58 | 0.14 | 0.06 | - | - | - | 0.90 | - |
| 6.22 | α-Phellandrene | - | 1.51 | 0.12 | 0.14 | 020 | 0.61 | - | 0.20 | - | 0.14 |
| 6.48 | α-Pinene | 12.62 | 11.45 | 12.97 | 9.17 | 24.68 | 20.13 | 6.11 | 40.68 | 18.15 | 13.73 |
| 6.94 | Camphene | 2.12 | 1.46 | 2.49 | 0.52 | 0.58 | 1.06 | - | 0.98 | 3.90 | 0.22 |
| 7.78 | Sabinene | 0.76 | 4.31 | 4.56 | 2.87 | 2.32 | 0.99 | 1.21 | 0.86 | 2.83 | 6.77 |
| 7.88 | β-Pinene | 3.82 | 3.07 | 4.38 | 1.15 | 8.64 | 4.13 | 0.48 | 7.60 | 4.19 | 0.94 |
| 8.40 | β-Myrcene | 8.40 | 0.97 | 0.67 | 4.45 | 0.91 | 23.80 | 57.79 | 1.08 | 4.08 | 5.47 |
| 8.86 | 1-Phellandrene | - | 4.64 | 0.80 | 4.60 | 2.12 | 3.84 | 3.79 | 0.92 | 0.81 | 8.35 |
| 9.33 | α-Terpinene | - | 4.66 | 3.60 | 3.76 | 3.11 | 2.71 | 0.90 | 1.13 | 0.72 | 1.73 |
| 9.69 | Para Cymene | 7.79 | 0.64 | 0.22 | 0.50 | 0.35 | 0.48 | 0.34 | 0.16 | 0.42 | 0.98 |
| 9.81 | β-Phellandrene | - | 6.48 | 4.93 | 5.79 | 11.35 | 6.48 | 3.44 | 8.03 | - | 6.61 |
| 9.89 | Limonene | 18.00 | - | - | - | - | - | - | - | - | - |
| 10.21 | Cis-Ocimene | - | 0.60 | 035 | 1.00 | 0.72 | - | 0.29 | 0.22 | 1.08 | 0.61 |
| 10.62 | Trans-β-Ocimene | - | 0.41 | 0.23 | 0.53 | 0.46 | - | 0.57 | - | 0.32 | 0.23 |
| 10.96 | Butanoic Acid | - | - | - | - | 0.55 | - | - | 1.07 | - | - |
| 11.03 | γ-Terpinene | - | 7.42 | 5.90 | 6.11 | 4.89 | 4.93 | 1.85 | 2.24 | 1.51 | 2.96 |
| 12.26 | α-Terpinolene | - | 2.48 | 2.08 | 2.35 | 2.59 | 2.08 | 0.63 | 227 | 0.64 | 1.04 |
| 12.49 | 2-Nonanone | - | 0.34 | 0.17 | 0.19 | 0.63 | 0.44 | 0.31 | 1.65 | - | - |
| 15.74 | Endobomeol | 6.90 | 0.12 | 0.34 | - | 0.24 | 0.32 | - | 0.65 | - | - |
| 16.20 | 1-4 Terpineol | 3.08 | 14.89 | 12.26 | 13.11 | 7.12 | 8.78 | 4.09 | 2.63 | 3.03 | 6.98 |
| 16.79 | α-Tapineol | 0.72 | 4.92 | 5.36 | 6.42 | 7.41 | 7.97 | 1.11 | 9.83 | 1.41 | 1.13 |
| 19.44 | Octanoid Acid | - | 0.18 | 0.13 | 0.22 | - | - | - | 0.54 | - | 0.17 |
| 21.04 | Bomyl Acetate | 28.19 | 2.12 | 1.77 | 0.17 | - | 1.94 | - | - | 0.98 | 0.28 |
| 21.42 | 2-Undecanone | 0.60 | 0.69 | 0.19 | 0.55 | 0.99 | 0.41 | 0.59 | 1.38 | 1.47 | 0.65 |
| 24.77 | α-Ylangene | - | 0.42 | 0.87 | 0.70 | - | - | 0.32 | - | 0.46 | 0.60 |
| 25.39 | β-Cubebene | - | - | 0.25 | 0.21 | - | - | - | - | - | 0.34 |
| 25.47 | β-Elemene | - | 0.15 | 0.19 | 0.23 | 0.11 | - | - | - | 0.52 | 0.51 |
| 26.61 | β-Caryophyllene | 1.63 | 2.79 | 3.21 | 3.24 | 4.02 | 0.44 | 0.81 | 1.18 | 3.91 | 1.70 |
| 27.85 | α-Cubebene | - | - | 0.05 | 0.24 | - | - | - | - | - | - |
| 27.96 | α-Humulene | - | 1.02 | 0.88 | 1.13 | 0.48 | 0.25 | 0.37 | - | 1.10 | 0.60 |
| 28.99 | α-Amorphene | 0.43 | 0.85 | 1.51 | 1.30 | 0.17 | 0.35 | 0.80 | 0.39 | 1.16 | 1.13 |
| 29.10 | Germacrene D | - | 4.71 | 6.82 | 5.00 | 1.37 | 2.06 | 2.76 | 1.23 | 8.36 | 8.32 |
| 29.66 | α-Elemene | - | 0.33 | 0.41 | 0.51 | 0.26 | - | - | 0.35 | 0.90 | 0.99 |
| 29.89 | α-Muurolene | - | 0.71 | 1.21 | 1.07 | 0.33 | - | 0.68 | 0.44 | 0.98 | 0.93 |
| 30.05 | Germacrene A | - | 0.14 | 0.18 | 0.30 | 0.16 | - | - | - | 0.44 | 0.68 |
| 30.42 | Butylated Hydroxy Toluene | - | 0.60 | 0.81 | 1.36 | 0.44 | 0.88 | 123 | 1.72 | 4.33 | 1.25 |
| 30.81 | δ-Cadinene | - | 2.82 | 4.80 | 4.40 | 1.11 | 1.32 | 3.28 | 1.74 | 4.44 | 3.99 |
| 33.20 | Caryophyllene Oxide | 2.53 | - | - | - | 0.12 | - | - | - | 0.83 | - |
| 34.79 | Cadina 1.4-Diene | - | 0.83 | 1.26 | 1.50 | 0.20 | - | 0.46 | - | 0.86 | 0.71 |
| 34.92 | γ-Eudesmol | - | 121 | 0.96 | 1.07 | 0.48 | 0.41 | 0.63 | 0.28 | - | 0.61 |
| 35.31 | α-Cadinol | - | 2.50 | 3.29 | 4.13 | 1.63 | 0.76 | 1.96 | 0.97 | 3.18 | 2.70 |
| 35.45 | α-Copaene | - | 0.75 | 1.03 | 1.29 | 0.49 | - | 0.53 | 0.31 | 1.22 | 0.81 |
| 35.58 | β-Eudesml | - | 0.47 | 0.31 | 0.41 | 0.19 | - | - | 0.20 | - | 0.46 |
| 35.80 | T-Cadinolo | 0.85 | 3.16 | 3.90 | 4.73 | 2.61 | 1.15 | 2.79 | 1.68 | 4.53 | 3.77 |
| 56.28 | Nonadecane | - | - | - | - | - | - | - | - | - | - |
| 61.56 | Octadecane | - | - | - | - | - | - | - | - | - | - |
| 67.79 | 4Chloro2Phenylandine | - | - | - | - | - | - | - | - | - | - |
| 67.96 | BenzenelMethoxy2,3,5Trimet | - | - | - | - | - | - | - | - | - | - |
| 69.78 | 4.12BisHydroxymethyl | - | - | - | - | - | - | - | - | - | - |

**Table 1b. P. terebintbus Composition**

| **RT*** | **Oil Composition** | | | | | **Leaves** | | | | | **Fruits** | | | **Galls** | | **Flowers** | **Branches** |
|---|---|---|---|---|---|---|---|---|---|---|---|---|---|---|---|---|---|
| | | **DM1Z** | **DM2S** | **DMF3** | **DMV2X** | **DMV5X** | **DM4a** | **DM4p** | **DM5a** | **DM4C** | **DMK** | **DMF2** | **DMG1** | **DM1P** | **DM2C** | **DM3C** | **DM2P** |
| 6.08 | Tricyclene | - | - | - | - | - | 0.25 | - | - | - | 0.30 | - | - | - | 0.48 | 0.45 | 0.18 |
| 6.22 | α-Phellandrene | - | - | - | - | - | - | - | - | - | - | - | - | - | 0.21 | - | 0.36 |
| 6.48 | α-Pinene | 35.63 | 12.17 | 41.03 | 11.61 | 14.09 | 60.99 | 10.04 | 10.99 | 18.67 | 31.63 | 8.69 | 54.19 | 42.16 | 28.90 | 26.43 | 28.72 |
| 6.94 | Camphene | 0.55 | 0.26 | 1.02 | - | - | 1.56 | 0.24 | 0.37 | 0.46 | 1.50 | 0.48 | 0.69 | 0.67 | 2.12 | 1.83 | 1.06 |
| 7.78 | Sabinene | - | 0.62 | - | 0.39 | - | 0.22 | 0.15 | 0.28 | - | 1.20 | 0.85 | 0.65 | 0.18 | 2.00 | 0.91 | 4.53 |
| 7.88 | β-Pinene | 1.12 | 1.14 | 6.17 | 1.39 | 0.71 | 1.87 | 0.32 | 1.48 | 3.27 | 7.44 | 1.39 | 3.68 | 1.81 | 6.40 | 5.85 | 4.07 |
| 8.40 | β-Myrcene | 1.57 | 1.68 | 1.37 | 1.82 | 2.03 | 1.98 | 1.41 | 1.60 | 2.90 | 1.21 | 1.11 | 1.05 | 1.42 | 1.68 | 0.97 | 1.67 |
| 8.86 | I-Phellandicne | 0.62 | 0.35 | 0.49 | 0.75 | 0.62 | 0.47 | 0.19 | 0,29 | 0.27 | 3.34 | 7.66 | 0.49 | 6.61 | 0.32 | 0.37 | 0.59 |
| 9.33 | α-Terpinene | - | 0.43 | 0.83 | 0.71 | - | 0.44 | - | 0.21 | 0.34 | 0.25 | 0.34 | - | - | 0.65 | 0.51 | 1.46 |
| 9.69 | Para Cymene | - | 0.11 | - | - | - | 0.17 | - | - | - | 0.38 | - | - | 0.18 | 0.20 | - | 0.49 |
| 9.81 | β-Phellandrene | - | - | - | - | - | - | - | - | - | - | - | - | - | - | - | - |
| 9.89 | Limonene | 27.45 | 50.70 | 6.09 | 21.04 | 60.47 | 6.16 | 1.23 | 8.03 | 60.24 | 12.59 | 32.85 | 13.97 | 31.07 | 28.76 | 23.03 | 6.56 |
| 10.21 | Cis-Ocimene | 19.63 | 15.64 | 10.93 | 20.97 | 3.86 | 6.31 | 63.60 | 54.19 | 0.96 | 6.79 | 17.99 | 1.89 | 0.37 | - | 3.88 | 13.44 |
| 10.62 | Trans-β-Ocimene | 5.49 | 4.45 | 3.80 | 6.04 | 1.17 | 1.86 | 18.29 | 16.46 | 0.38 | 1.66 | 5.01 | 0.40 | - | - | 1.22 | 3.33 |
| 10.96 | Butanoic Acid | - | - | - | - | - | - | - | - | - | - | - | - | - | - | - | - |
| 11.03 | γ-Terpinene | 0.22 | 0.69 | 0.48 | 0.62 | - | 0.49 | 0.17 | 0.34 | 0.60 | 0.33 | 0.42 | 0.12 | - | 1.08 | 0.77 | 2.46 |
| 12.26 | α-Terpinolene | 0.92 | 0.93 | 17.07 | 16.59 | 4.90 | 7.13 | 0.38 | 0.46 | 1.76 | 0.44 | 1.27 | 0.26 | 0.28 | 0.57 | 2.99 | 1.08 |
| 12.49 | 2-Nonanone | - | - | - | - | - | - | - | - | - | - | - | - | - | - | - | - |
| 15.74 | Endobomeol | - | - | - | - | - | - | - | - | - | - | - | - | - | - | - | - |
| 16.20 | 1-4 Terpineol | 0.3 | 1.63 | 0.29 | 1.86 | 1.21 | 0.29 | 0.35 | 0.50 | 0.46 | 1.00 | 0.87 | 0.18 | 0.13 | 2.86 | 1.55 | 8.91 |
| 16.79 | α-Terpineol | 1.63 | 4.05 | 5.22 | 1.92 | 1.18 | 2.23 | 1.35 | 1.22 | 2.87 | 2.43 | 0.93 | 0.35 | 1.36 | 1.69 | 1.45 | 2.26 |
| 19.44 | Octanoid Acid | - | - | - | - | - | - | - | - | - | - | - | - | - | - | - | - |
| 21.04 | Bomyl Acetate | - | - | 0.47 | - | - | - | - | - | - | 0.64 | 0.35 | - | 0.18 | - | - | 0.23 |
| 21.42 | 2-Undecanone | - | - | - | - | - | - | - | - | - | - | - | - | - | - | - | - |
| 24.77 | α-Ylangene | 0.23 | 0.16 | - | - | - | - | - | - | - | 4.51 | 0.96 | 0.15 | 0.19 | - | 2.14 | 0.51 |
| 25.39 | β-Cubebene | - | - | - | - | - | - | - | - | - | 1.60 | 0.40 | - | - | - | 0.37 | 0.19 |
| 25.47 | β-Elemene | - | - | - | - | - | - | - | - | - | - | - | - | - | - | - | - |
| 26.61 | β-Caryophyllene | 3.23 | 1.38 | 2.75 | 4.72 | 2.00 | 3.86 | 0.62 | 1.29 | 3.43 | 4.77 | 1.64 | 14.33 | 0.94 | 0.97 | 8.05 | 1.06 |
| 27.85 | α-Cubebene | - | - | - | - | - | - | - | - | - | 0.26 | 0.42 | - | - | - | - | 0.74 |
| 27.96 | α-Humulene | 0.47 | 0.33 | - | 0.86 | 1.07 | 1.21 | - | - | 1.46 | 1.47 | 0.55 | 2.15 | 0.33 | 0.65 | 1.24 | 0.22 |
| 28.99 | α-Amorphene | - | 0.24 | - | - | - | - | 0.17 | - | 0.30 | 0.55 | 0.24 | - | 0.15 | 0.43 | 0.48 | 0.77 |
| 29.10 | Germacrene D | 0.23 | 0.62 | - | 1.56 | 1.40 | - | 0.14 | 0.45 | - | 2.24 | 9.42 | 2.91 | 7.08 | 5.87 | 2.44 | 2.91 |
| 29.66 | α-Elemene | - | - | - | - | - | - | - | - | - | 0.18 | - | - | - | - | - | 0.46 |
| 29.89 | α-Muurolene | - | - | - | - | - | - | - | - | - | 0.25 | - | - | - | 0.33 | 0.27 | 0.47 |
| 30.05 | Germacrene A | - | - | - | - | - | - | - | - | - | - | - | - | 0.14 | - | - | - |
| 30.42 | Butylated Hydroxy Toluene | 0.32 | 0.77 | 0.37 | 3.34 | 2.36 | 0.62 | 0.46 | 0.45 | 0.55 | 0.58 | 0.46 | 030 | 0.45 | 1.56 | 1.58 | 1.82 |
| 30.81 | δ-Cadinene | 0.44 | 0.67 | - | 1.32 | 0.92 | 0.31 | 0.31 | 0.63 | 0.61 | 6.42 | 2.33 | 0.31 | 0.55 | 1.22 | 3.61 | 3.51 |
| 33.20 | Caryophyllene Oxide | - | 0.23 | - | - | - | 0.27 | - | - | - | 0.40 | - | 0.39 | - | - | 0.75 | - |
| 34.79 | Cadina 1,4-Diene | - | - | - | - | - | - | - | - | - | 0.31 | 0.17 | - | - | - | 0.36 | 0.31 |
| 34.92 | γ-Eudesmol | - | - | - | - | - | - | - | - | - | - | - | - | 0.70 | - | - | - |
| 35.31 | α-Cadinol | - | - | - | - | - | - | - | - | - | 0.26 | - | - | 0.21 | 0.81 | 0.73 | 0.53 |
| 35.45 | α-Copaene | - | - | - | - | - | - | - | - | - | 0.39 | 0.55 | - | - | 0.25 | 0.19 | - |
| 35.58 | β-Eudesmol | - | - | - | - | - | - | - | - | - | - | - | - | 0.21 | - | - | - |
| 35.80 | T-Cadinolo | - | 0.17 | - | - | - | - | - | - | - | 0.31 | 0.65 | - | 0.30 | 1.14 | 0.87 | 0.36 |
| 56.28 | Nonadecane | - | - | - | - | - | - | - | - | - | - | - | - | - | 2.20 | 1.04 | - |
| 61.56 | OCtadecane | - | - | - | 0.47 | - | - | - | - | 0.25 | - | - | - | - | 1.99 | 0.71 | - |
| 67.79 | 4Chloro2Phenylaniline | - | - | - | - | - | - | - | - | - | - | - | - | - | - | - | - |
| 67.96 | BenzenelMethoxy2,3,5Trimet | - | - | - | - | - | - | - | - | - | - | - | - | - | - | - | - |
| 69.78 | 4,12BisHydroxymethyl | - | - | - | - | - | - | - | - | - | - | - | - | - | - | - | - |

**Table 1c. P. Vera (DM1S) and P. integerrima (DMNP)Composition**

| **RT*** | **Oil Composition** | **DM1S Nuts** | **DMNP Galls** |
|---|---|---|---|
| 6.08 | Tricyclene | - | - |
| 6.22 | α-Phellandrene | - | - |
| 6.48 | α-Pinene | 0.99 | 14.41 |
| 6.94 | Camphene | - | 0.94 |
| 7.78 | Sabinene | - | 2.51 |
| 7.88 | β-Pinene | - | 5.62 |
| 8.40 | β-Myrcene | - | 2.81 |
| 8.86 | 1-Phellandrene | - | 0.56 |
| 9.33 | α-Terpinene | - | 0.80 |
| 9.69 | Para Cymene | - | 2.09 |
| 9.81 | β-Phellandrene | - | - |
| 9.89 | Limonene | 1.54 | 5.94 |
| 10.21 | Cis-Ocimene | - | - |
| 10.62 | Trans-β-Ocimene | - | - |
| 10.96 | Butanoic Acid | - | - |
| 11.03 | γ-Terpinene | - | 1.52 |
| 12.26 | α-Terpinolene | - | 0.58 |
| 12.49 | 2-Nonanone | - | - |
| 15.74 | Endobomeol | - | 3.12 |
| 16.20 | 1-4 Terpineol | 2.13 | 29.52 |
| 16.79 | α-Terpineol | 2.56 | 14.08 |
| 19.44 | Octanoid Acid | - | - |
| 21.04 | Bornyl Acetate | - | 4.55 |
| 21.42 | 2-Undecanone | - | - |
| 24.77 | α-Ylangene | - | - |
| 25.39 | β-Cubebene | - | - |
| 25.47 | β-Elemene | - | - |
| 26.61 | β-Caryophyllene | 0.60 | 2.81 |
| 27.85 | α-Cubebene | - | - |
| 27.96 | α-Humulene | - | - |
| 28.99 | α-Amorphene | - | - |
| 29.10 | Germacrene D | 0.62 | - |
| 29.66 | α-Elemene | - | - |
| 29.89 | α-Muurolene | - | - |
| 30.05 | Germacrene A | - | - |
| 30.42 | Butylated Hydroxy Toluene | 15.74 | - |
| 30.81 | δ-Cadinene | 0.66 | - |
| 33.20 | Caryophyllene Oxide | - | - |
| 34.79 | Cadina 1,4-Diene | - | - |
| 34.92 | γ-Eudesmol | - | - |
| 35.31 | α-Cadinol | - | - |
| 35.45 | α-Copaene | - | - |
| 35.58 | β-Eudesmol | - | - |
| 35.80 | T-Cadinolo | - | - |
| 56.28 | Nonadecane | - | - |
| 61.56 | Octadecane | - | - |
| 67.79 | 4Chloro2Phenylaniline | 32.88 | - |
| 67.96 | BenzenelMethoxy2,3,5Trimet | 25.63 | - |
| 69.78 | 4,12BisHydroxymethyl | 4.29 | - |

| | | | |
|---|---|---|---|
| RT= Retention time | | | |

### RESULTS OF THE BIOLOGICAL TESTS

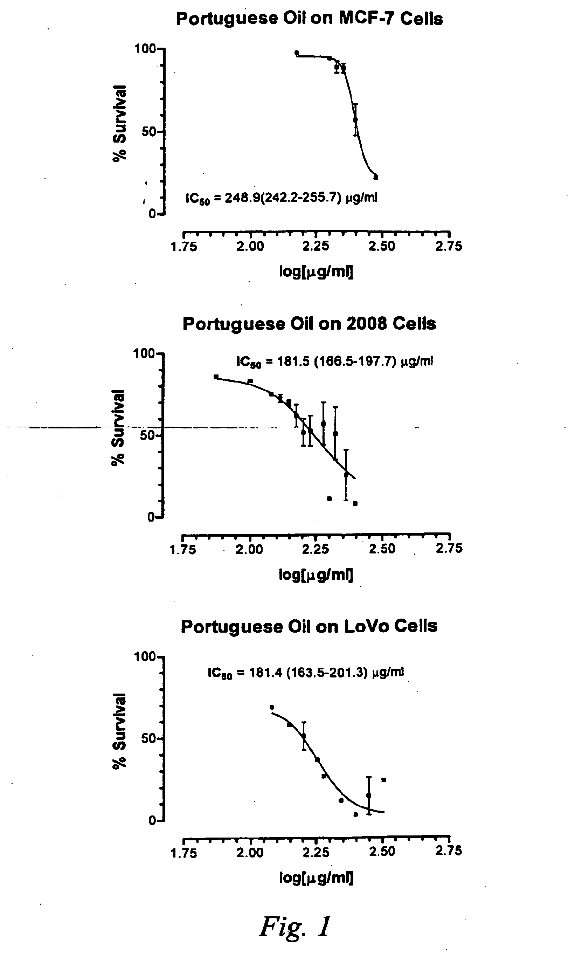

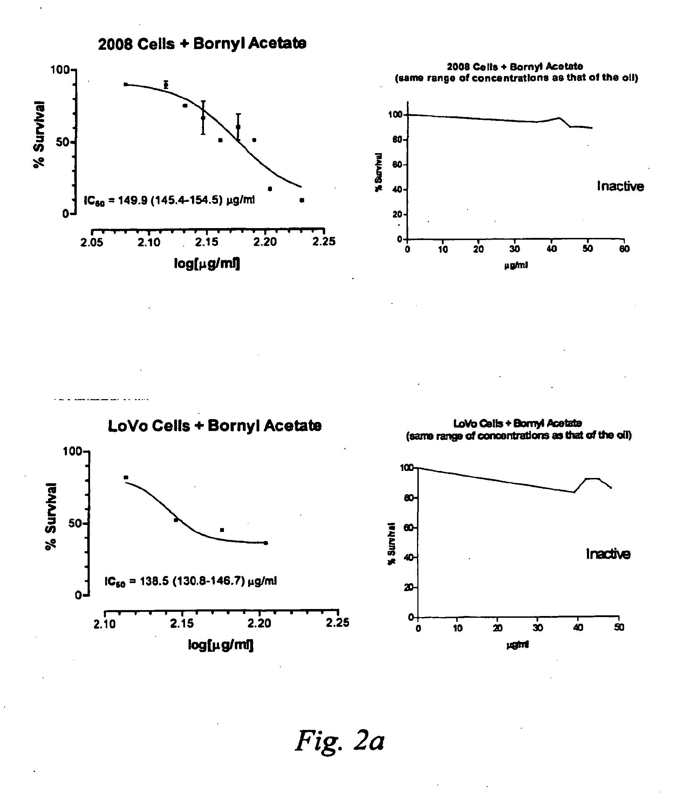

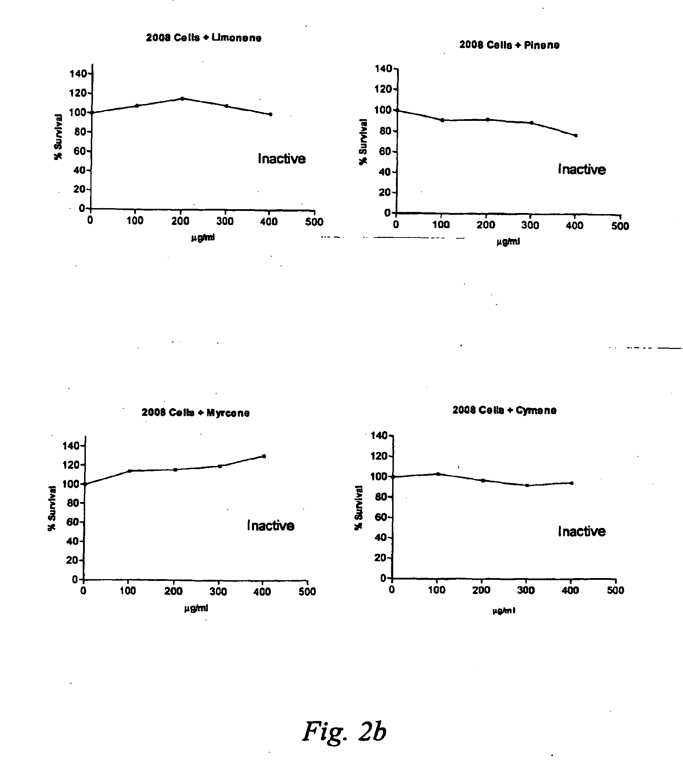

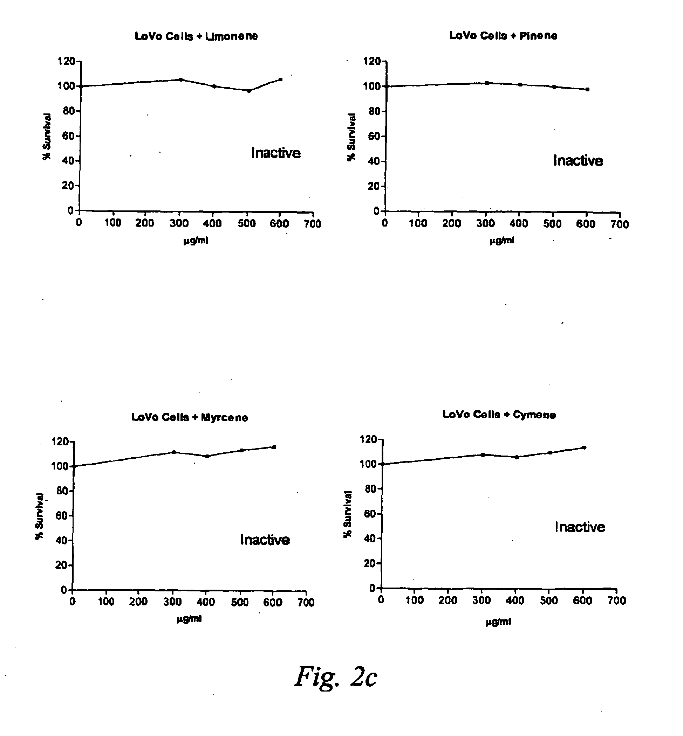

Following are some examples of results obtained evaluating the cytotoxicity of several essential oils against the selected tumor cell lines (MCF-7, LoVo and 2008) and showed that the oil from leaves of Portuguese *P lentiscus* (see Table 1a for composition) was active in inducing a cytotoxic effect (Fig. 1) with IC₅₀ of 248 (242.3-255.7) µg/ml on MCF-7, 181.5 (166.5-197.7) µg/ml on 2008 and 181.4 (163.7-201.3) µg/ml on LoVo cells. The cytotoxic effect was also assayed using some of the single components of the oil and the only active tested component, in our experimental conditions, was Bornyl Acetate (Fig. 2), but when this compound was utilized at the equivalent concentration of the oil it resulted inactive (Fig. 2). Among the single components tested, limonene, which has been reported in literature as having antitumoral effect, did not prove active in our experimental conditions.

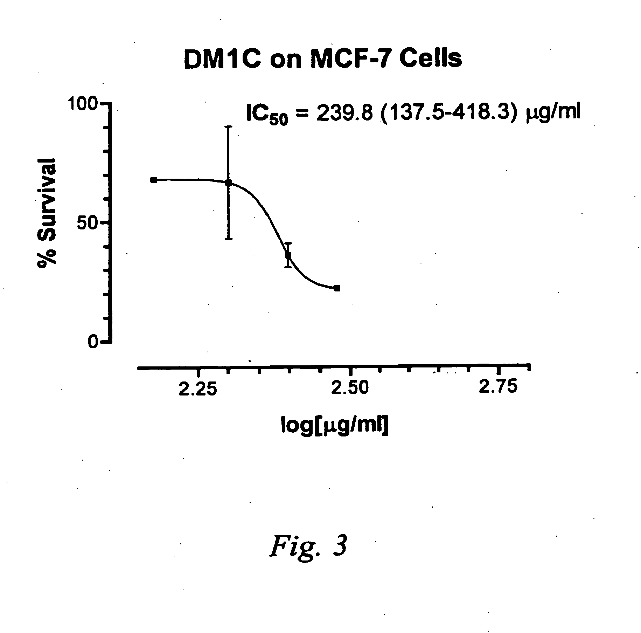

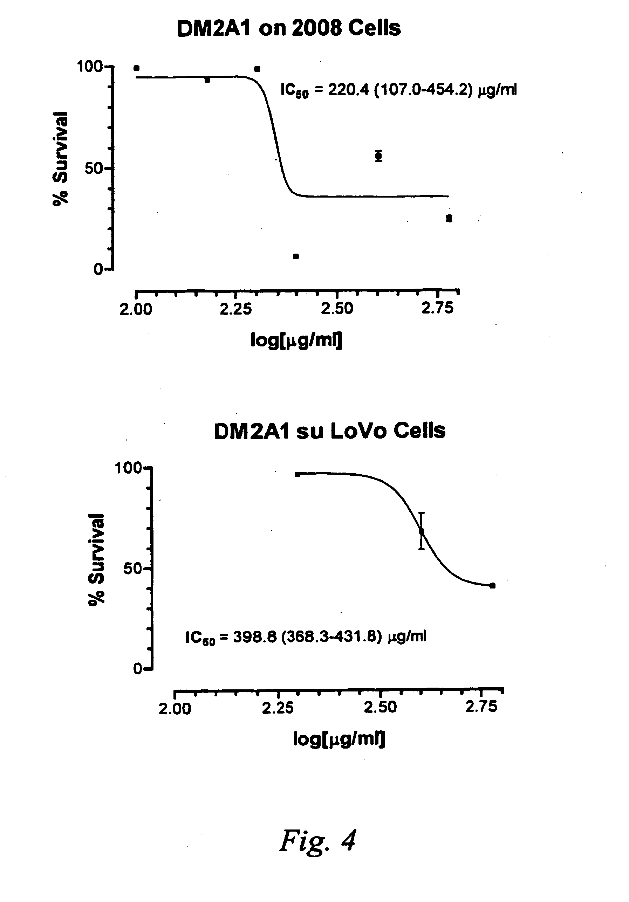

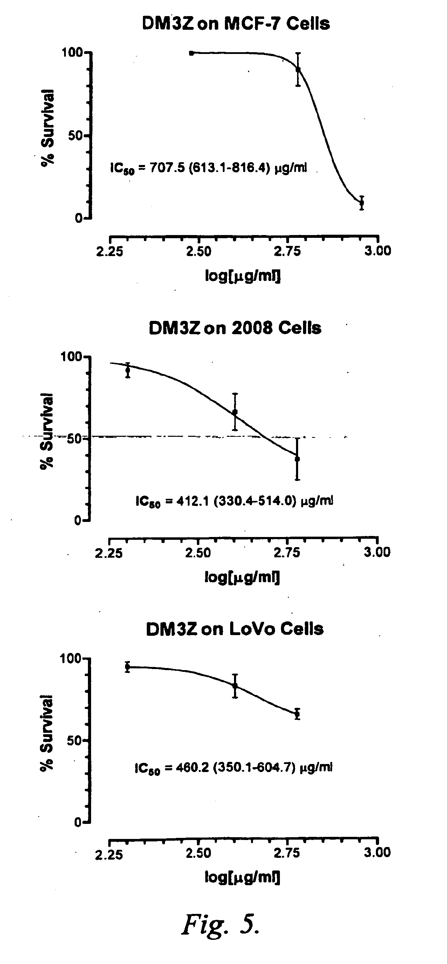

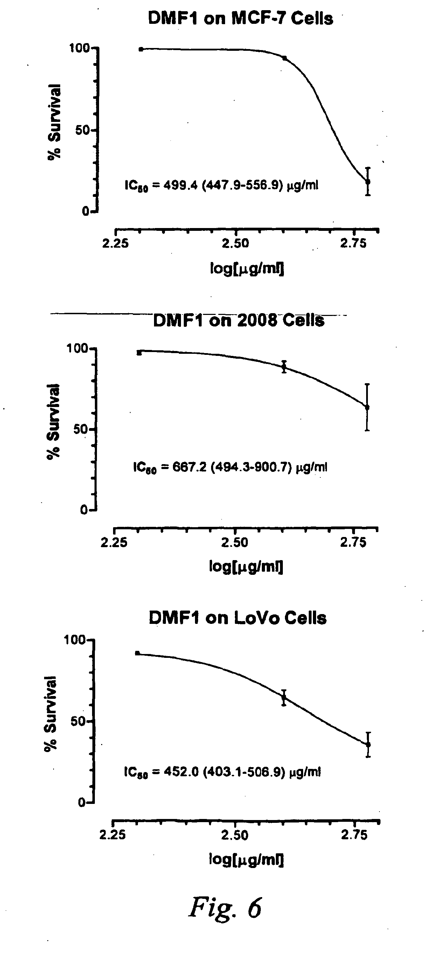

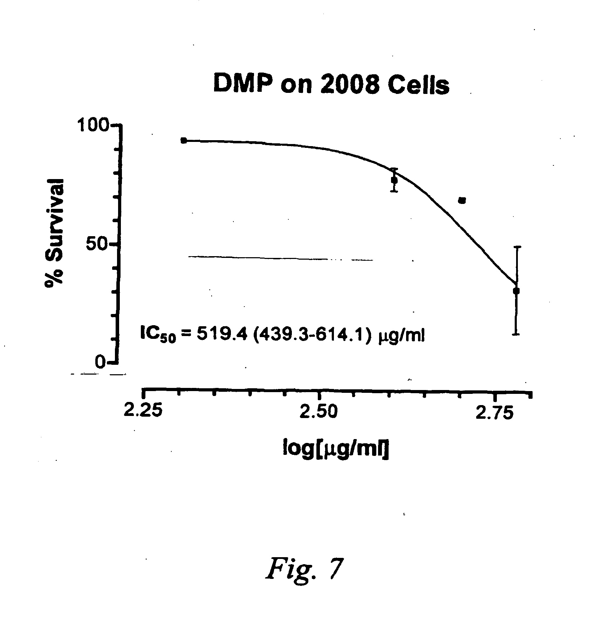

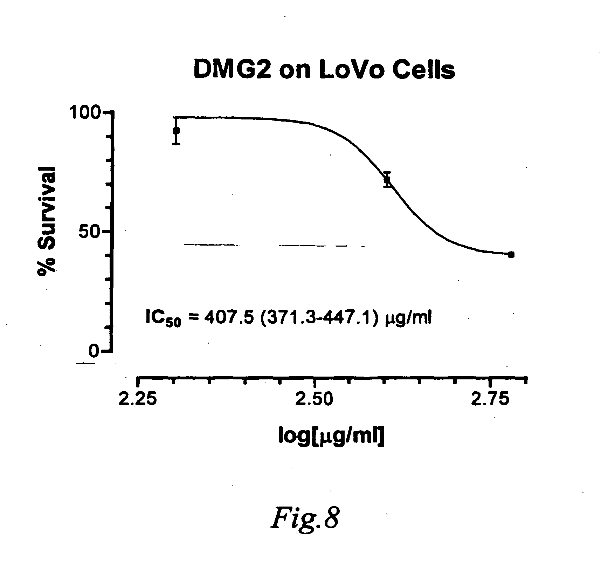

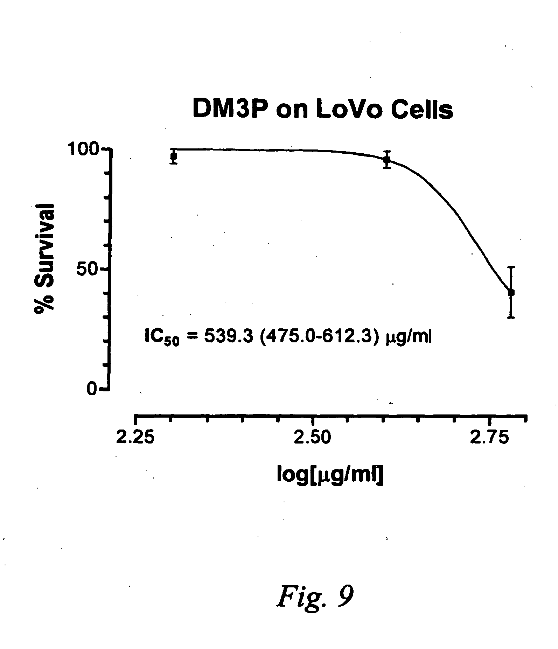

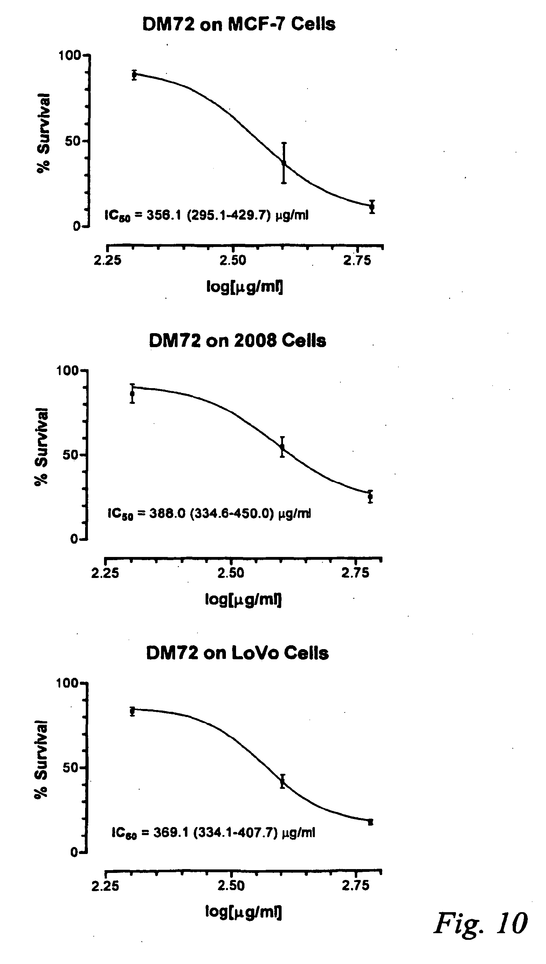

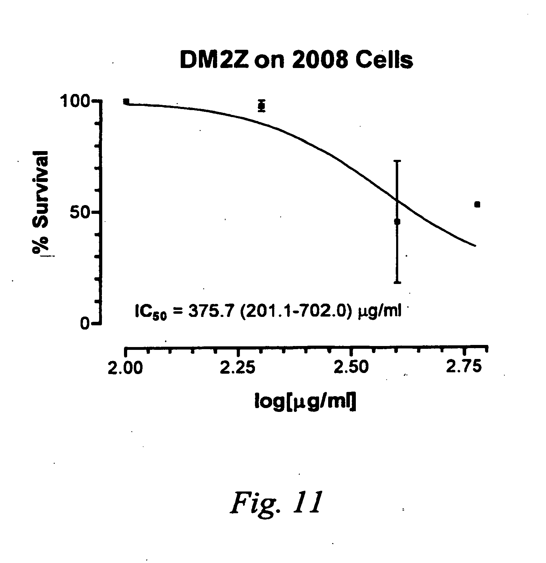

Other results showed that *Pistacia* essential oils were able to reduce cell growth. In particular, DM1C (oil extracted from one sample of *P. lentiscus* leaves) resulted active in inducing cytotoxicity in MCF-7 cell lines with IC₅₀ of 239.8 (137.5-418.3)µg/ml (Fig.3). DM2A1 (oil extracted from one sample of *P. lentiscus* leaves) resulted active in inducing cytotoxicity in both 2008 and LoVo cell lines with IC₅₀ of 220.4 (107.0-454.2) µg/ml and 398.8 (368.3-431.8) µg/ml, respectively (Fig.4). DM3Z (oil extracted from one sample of *P. lentiscus* leaves) resulted active in inducing cytotoxicity in both MCF-7, 2008 and LoVo cell lines with IC₅₀ of 707.5 (613.1-816.4) µg/ml, 412.1 (330.4-514.0) µg/ml and 460.2 (350.1-604.7) µg/ml, respectively (Fig.5). DMF1 (oil extracted from one sample of *P. lentiscus* leaves) resulted active in inducing cytotoxicity in both MCF-7, 2008 and LoVo cell lines with IC₅₀ of 499.4 (447.9-556.9) µg/ml, 667.2 (494.3-900.7) µg/ml and 452.0 (403.1-506.9) µg/ml, respectively (Fig.6). DMP (oil extracted from one sample of *P. lentiscus* berries) resulted active in inducing cytotoxicity in 2008 cell lines with IC₅₀ of 519.4 (439.3-614.1) µg/ml (Fig.7). DMG2 (oil extracted from one sample of *P. lentiscus* berries) resulted active in inducing cytotoxicity in LoVo cell lines with IC₅₀ of 407.5 (371.3-447.1) µg/ml (Fig.8). DM3P (oil extracted from one sample of *P. lentiscus* berries) resulted active in inducing cytotoxicity in LoVo cell lines with IC₅₀ of 539.3 (475.0-621.3) µg/ml (Fig.9). DM72 (oil extracted from one sample of *P*. *lentiscus* branches) resulted active in inducing cytotoxicity in both MCF-7, 2008 and LoVo cell lines with IC₅₀ of 356.1 (295.1-429.7) µg/ml, 388.0 (334.6-450.0) µg/ml and 369.1 (334.1-407.7) µg/ml, respectively (Fig.10). DM2Z (oil extracted from one sample of *P. lentiscus* branches) resulted active in inducing cytotoxicity in 2008 cell lines with IC₅₀ of 375.7 (201.1-702.0) µg/ml (Fig.11).

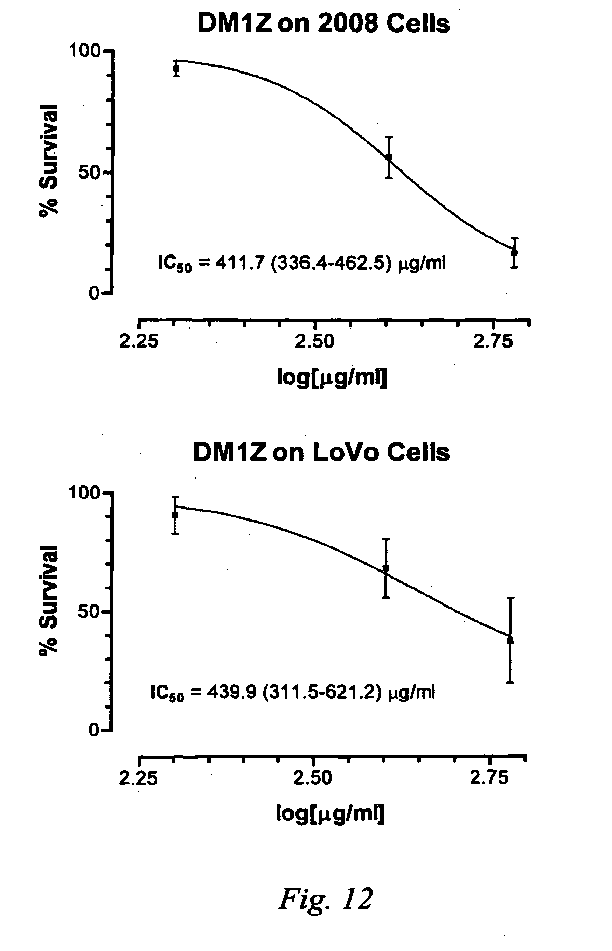

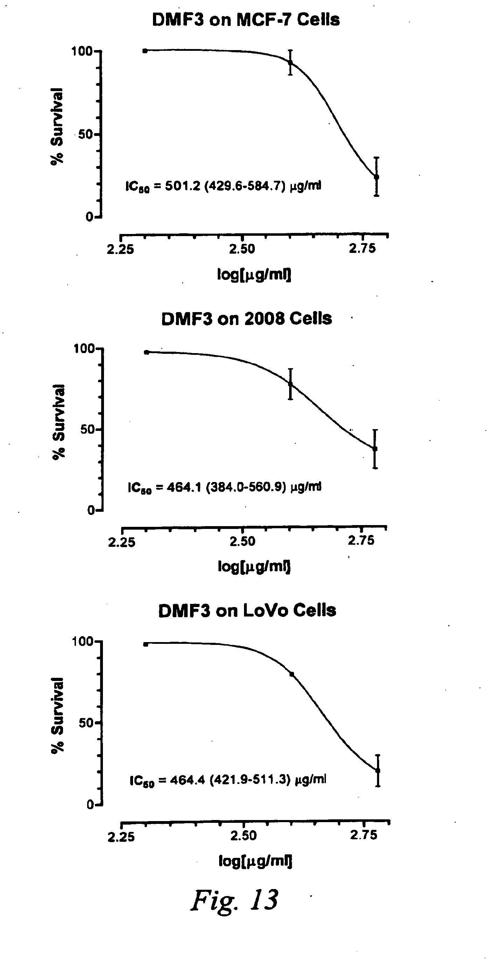

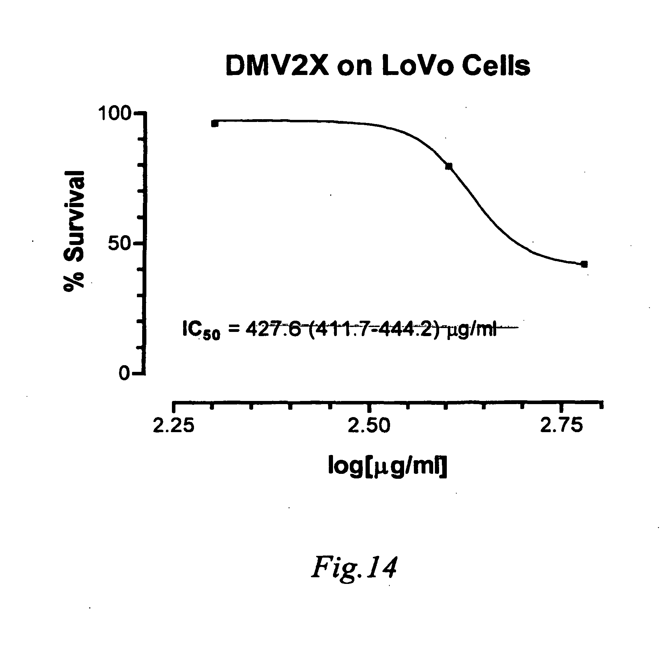

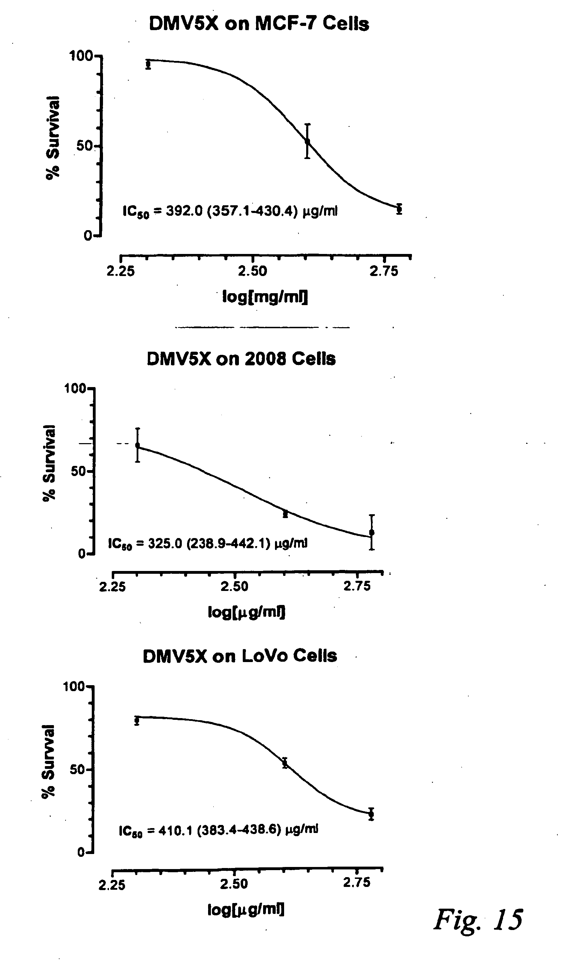

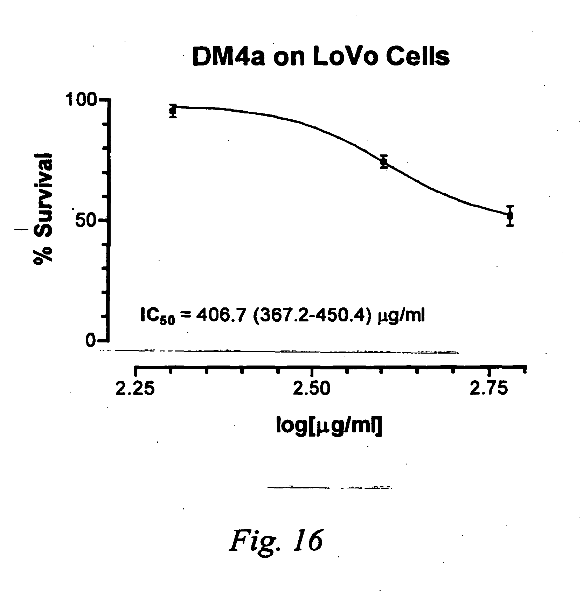

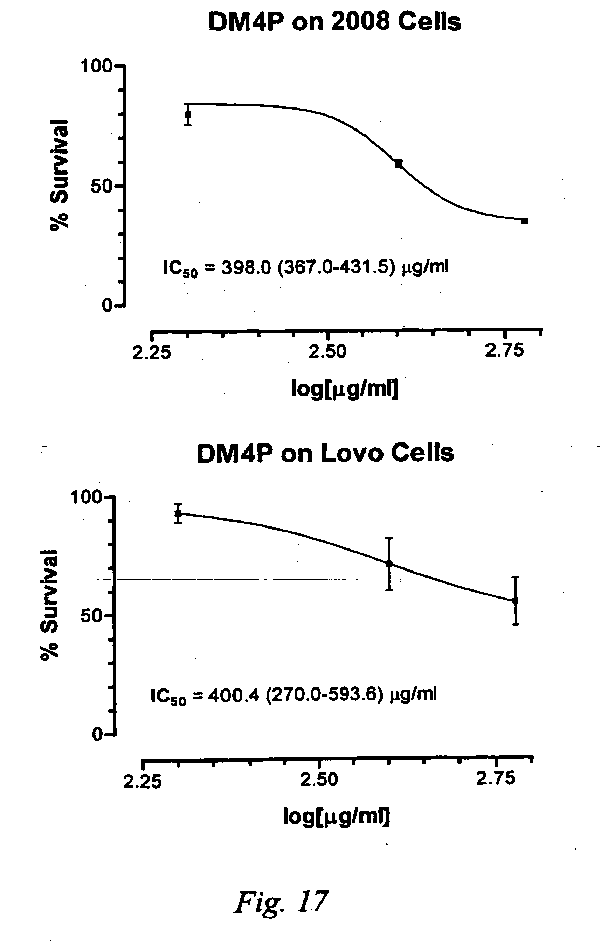

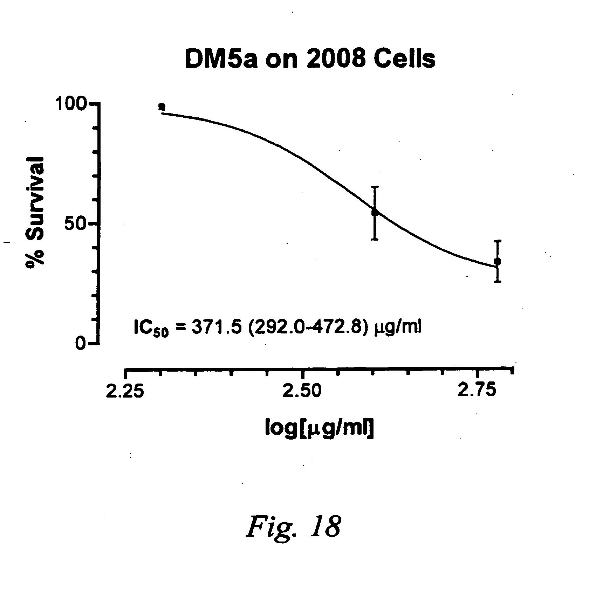

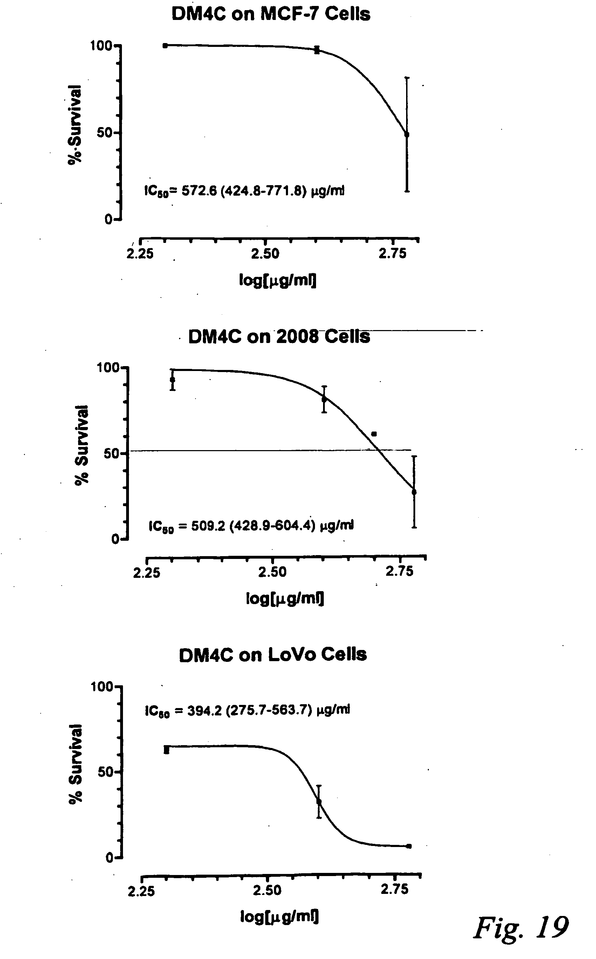

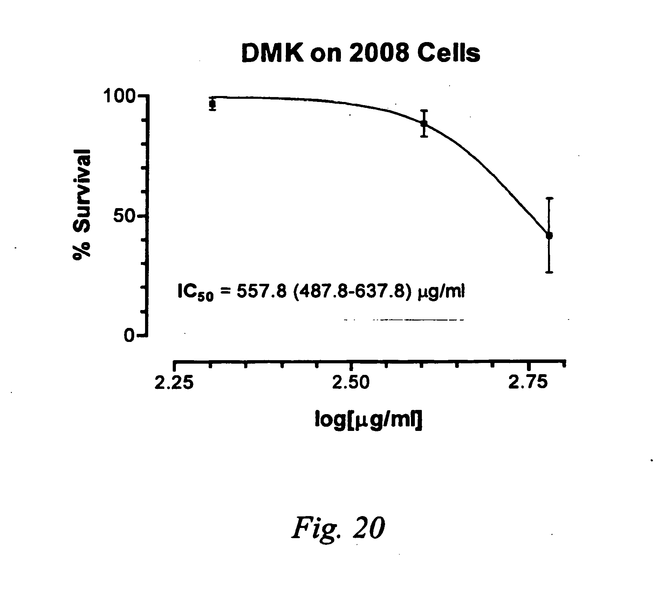

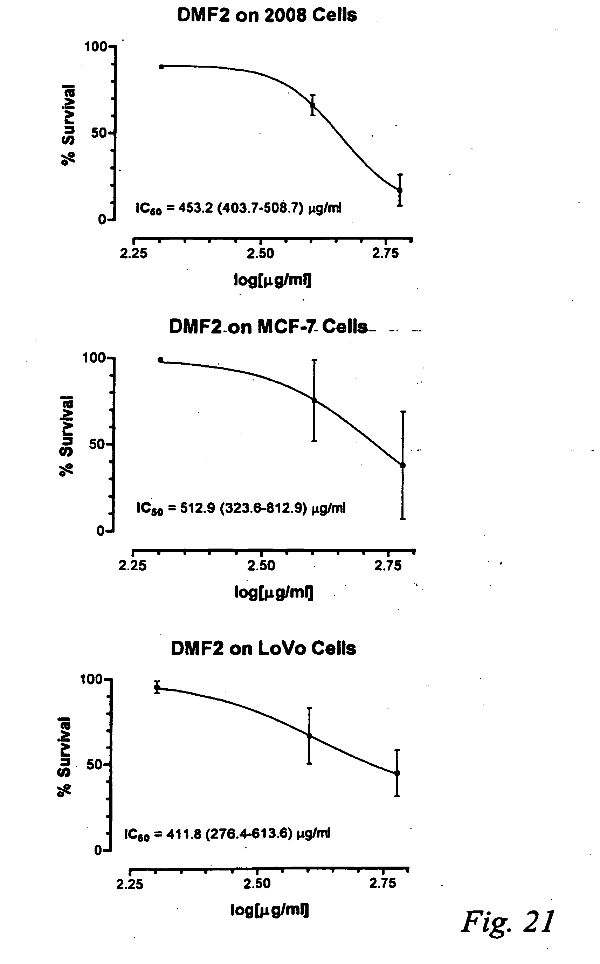

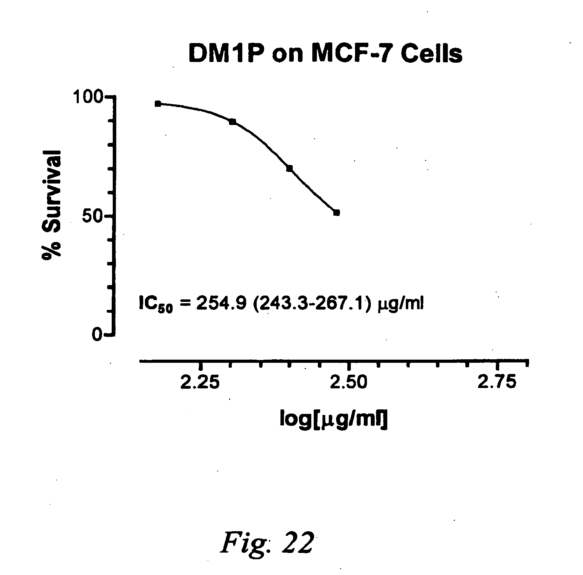

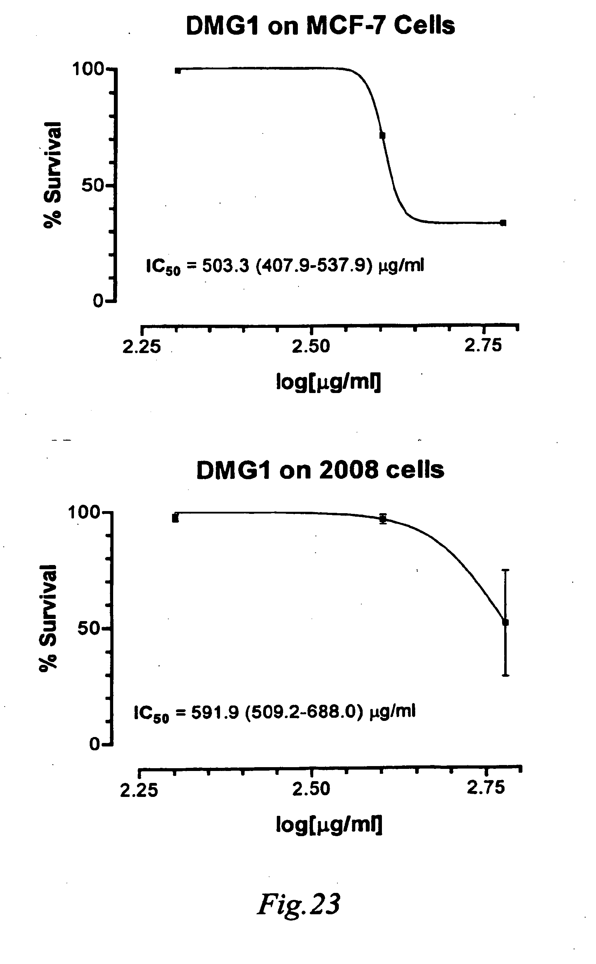

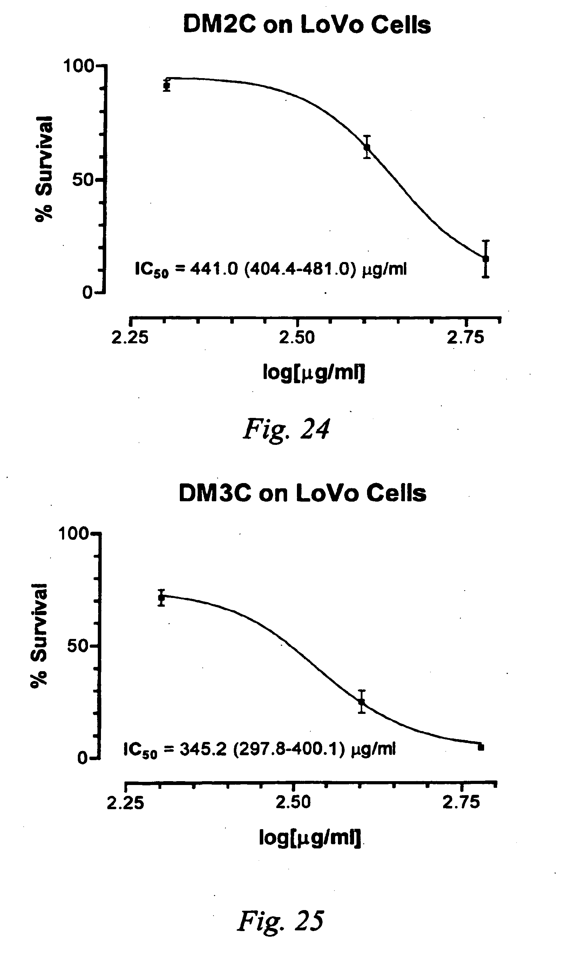

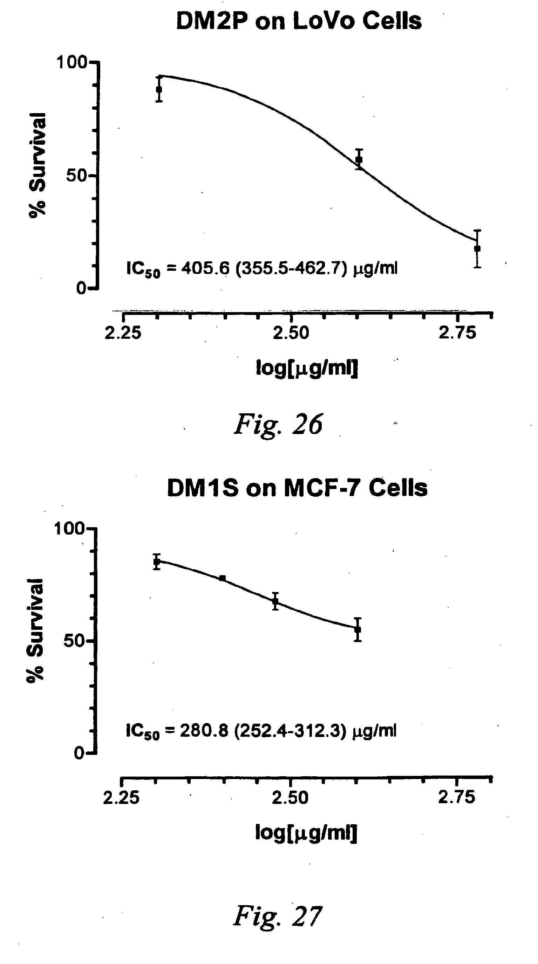

DM1Z (oil extracted from one sample of *P. terebinthus* leaves) resulted active in inducing cytotoxicity in both 2008 and LoVo cell lines with IC₅₀ of 411.7 (336.4-462.5) µg/ml and 439.9 (311.5-621.2) µg/ml, respectively (Fig. 12). DMF3 (oil extracted from one sample of *P. terebinthus* leaves) resulted active in inducing cytotoxicity in both MCF-7, 2008 and LoVo cell lines with IC₅₀ of 501.2 (429.6-584.7) µg/ml, 464.1 (384.0-560.9) µg/ml and 464.4 (421.9-511.3) µg/ml, respectively (Fig.13). DMV2X (oil extracted from one sample of *P. terebinthus* leaves) resulted active in inducing cytotoxicity in LoVo cell lines with IC₅₀ of 427.6 (411.7-444.2) µg/ml (Fig.14). DMV5X (oil extracted from one sample of *P. terebinthus* leaves) resulted active in inducing cytotoxicity in both MCF-7, 2008 and LoVo cell lines with IC₅₀ of 392.0 (357.1-430.4) µg/ml, 325.0 (238.9-442.1) µg/ml and 410.1 (383.4-438.6) µg/ml, respectively (Fig.15). DM4a (oil extracted from one sample of *P. terebinthus* leaves) resulted active in inducing cytotoxicity in LoVo cell lines with IC₅₀ of 406.7 (367.2-450.4) µg/ml (Fig.16). DM4p (oil extracted from one sample of *P. terebinthus* leaves) resulted active in inducing cytotoxicity in both 2008 and LoVo cell lines with IC₅₀ of 398.0 (367.0-431.5) µg/ml and 400.4 (270.0-593.6) µg/ml, respectively (Fig. 17). DM5a (oil extracted from one sample of *P. terebinthus* leaves) resulted active in inducing cytotoxicity in 2008 cell lines with IC₅₀ of 371.5 (292.0-472.8) µg/ml (Fig.18).DM4C (oil extracted from one sample of *P. terebinthus* leaves) resulted active in inducing cytotoxicity in both MCF-7, 2008 and LoVo cell lines with IC₅₀ of 572.6 (424.8-771.8) µg/ml, 509.2 (428.9-604.4) µg/ml and 394.2 (275.7-563.7) µg/ml, respectively (Fig.19). DMK (oil extracted from one sample of *P. terebinthus* berries) resulted active in inducing cytotoxicity in 2008 cell lines with IC₅₀ of 557.8 (487.8-637.8) µg/ml (Fig.20). DMF2 (oil extracted from one sample of *P. terebinthus* berries) resulted active in inducing cytotoxicity in MCF-7, 2008 and LoVo cell lines with IC₅₀ of 512.9 (323.6-812.9) µg/ml, 453.2 (403.7-508.7) µg/ml and 411.8 (276.4-613.6) µg/ml, respectively (Fig.21). DM1P (oil extracted from one sample of *P. terebinthus* galls) resulted active in inducing cytotoxicity in MCF-7 cell lines with IC₅₀ of 254.9 (243.3-267.1) µg/ml (Fig.22). DMG1 (oil extracted from one sample of *P. terebinthus* galls) resulted active in inducing cytotoxicity in MCF-7 and 2008 cell lines with IC₅₀ of 503.3 (470.9-537.9) µg/ml and 591.9 (509.2-688.0) µg/ml, respectively (Fig.23). DM2C (oil extracted from one sample of *P. terebinthus* flowers) resulted active in inducing cytotoxicity in LoVo cell lines with IC₅₀ of 441.0 (404.4-481.0) µg/ml (Fig.24). DM3C (oil extracted from one sample of *P. terebinthus* flowers) resulted active in inducing cytotoxicity in LoVo cell lines with IC₅₀ of 345.2 (297.8-400.1) µg/ml (Fig.25). DM2P (oil extracted from one sample of *P. terebinthus* branches) resulted active in inducing cytotoxicity in LoVo cell lines with IC₅₀ of 405.6 (355.5-462.7) µg/ml (Fig.26).

DM1S (oil extracted from one sample of *P. vera* nuts) resulted active in inducing cytotoxicity in MCF-7 cell lines with IC₅₀ of 280.8 (252.4-312.3) µg/ml (Fig.27).

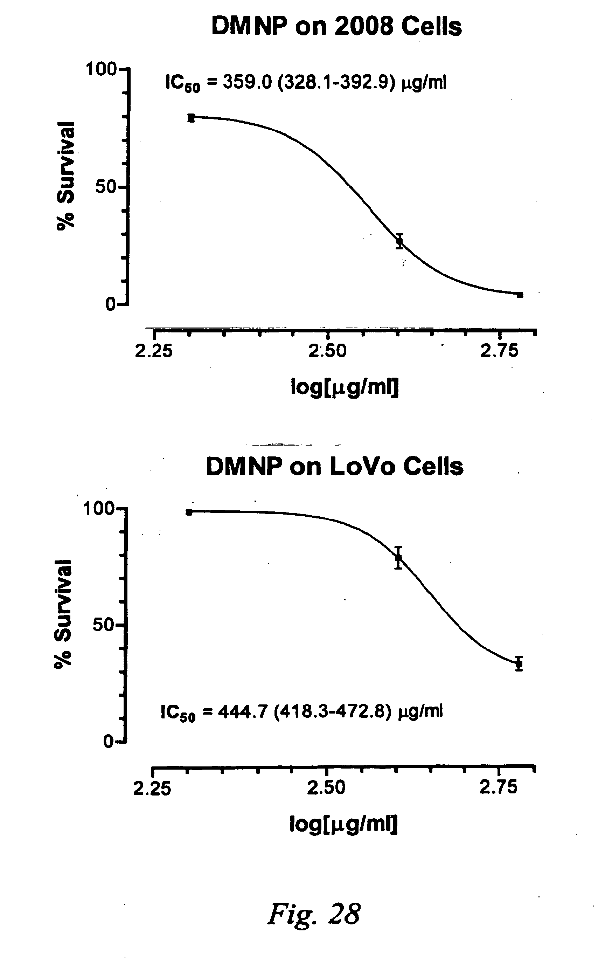

DMNP (oil extracted from one sample of *P. integerrima* galls) resulted active in inducing cytotoxicity in both 2008 and LoVo cell lines with IC₅₀ of 359.0 (328.1-392.9) µg/ml and 444.7 (418.3-472.8) µg/ml, respectively (Fig.28).

Only DM1S and DMNP are oils used in the claimed invention.

The activities of DM2A1, DM1Z, DM3Z, as well as that of the oil from Portugal were also tested with two cytofluorimetric assays to analyse the nature of cell death (Annexin V plus Propidium Iodide; NO production), which resulted mostly in apoptotic death for all oils.

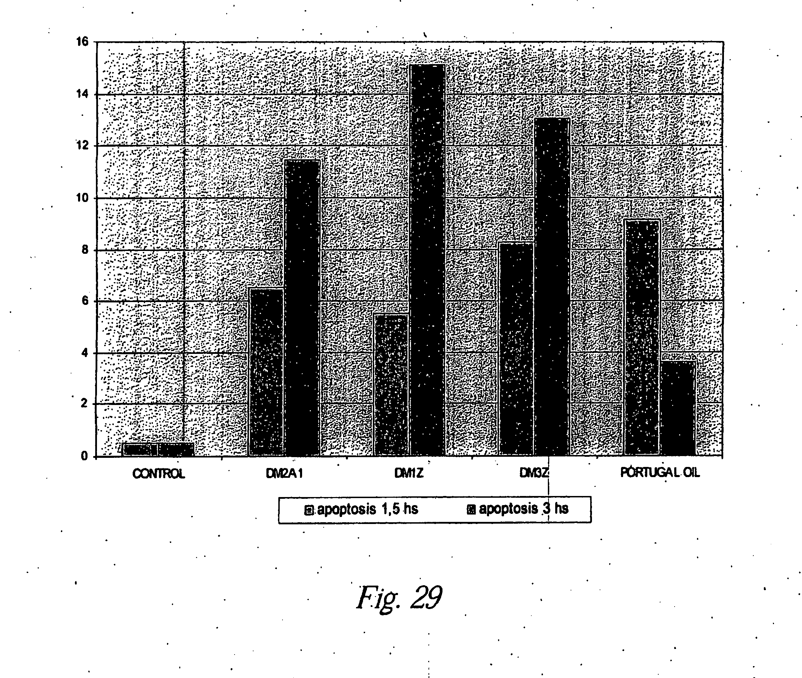

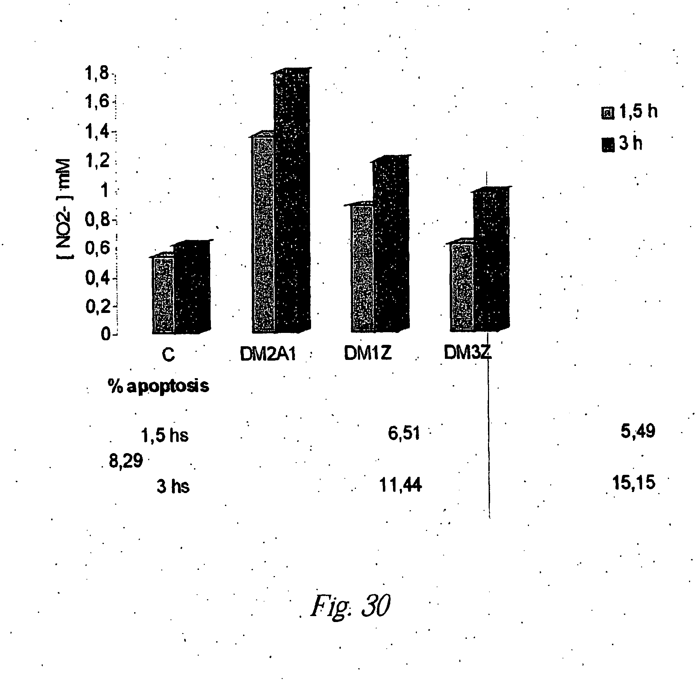

The cytofluometric tests indicated a cytotoxic effect with the same oils Fig. 29-30.

The results obtained evaluating the nitrite concentration in the culture medium of 2008 cells treated for 1.5 and 3 h with DM2A1, DM1Z and DM3Z indicated the activation of apoptotic mechanisms. It should be pointed out that these results were in accordance also to the cytotoxicity studies, indeed increasing the NO detected in medium, increasing the cytotoxic effect: DM2A1 was the most cytotoxic oil on 2008 cells and was able to induce the most production of NO.

### . REFERENCES

➢ Boelens M.H., Jemenez R. Chemical composition of the essential oil from the gum and various parts of Pistacia lentiscus L. (Mastic Gum Tree). Flav.Fragr.J., 6:271-275,1991.
➢ Buffoni F. La natura come fonte inesauribile di farmaci. Acta Phytotherapeutica., 2,3-6,1996.
➢ Buil P. et al. Contribution à la connaissance de la composition chimique de l'essence de lentisque de Provence. Riv. Ital. EPPOS Cosmet. Aerosol., 56:245-252,1975.
➢ Calabro G., Curro P. Costituenti degli oli essenziali Nota IV. Essenza di lentisco. Essence Deriv.Agrum., 44:82-92,1974.
➢ Castola V. et al. Intraspecific chemical variabilità of the essential oil of Pistacia lentiscus L. from Corsica. Biochem. System. Ecol., 28:79-88,2000.
➢ Chander S.K., Lansdown A.G.B., Luqmani Y.A., Gomm J.J., Coope R.C., Gould M.N. & Coope R.C. Gould M.N. & Coombes R.C. Effectiveness of combined limonene and 4-hydroxyandrostenedione in the treatment of NMU-induced rat mammary tumors. Br. J. Cancer., 69:879-882.
➢ Colombini M.P. et al. Characterization of the balm of an Egyptian mummy from the seventh century B.C. Studies in Conservation., 45:19-29,2000.
➢ Conner D.E., Beuchat L.R. Sensitivity of heat stressed yeasts to essential oils of plants. Appl. Environ. Microbiol., 47:229-233,1984.
➢ Cragg G.M. et al. Natural products in drug discovery and development. J. Nat. Prod., 60:52-60,1997.
➢ Cragg G.M., Newman D.J. Discovery and development of antineoplastic agents from natural sources. Cancer Invest., 17:153-163,1999.
➢ Croteau R. Biosynthesis and catabolism of monoterpenoids. Chem. Rev., 87:929-954,1987.
➢ Crowell P.L., Kennan W.S., Haag J.D., Ahmad S., Vedejs E. & Gould M.N. Chemoprevention of mammary carcinogenesis by hydroxylated derivatives of d-limonene. Carcinogenesis., 13:1261-1264,1992.
➢ Crowell P.L., Elson, Bailey, H.H., C.E., Elegbede A., Haag J.H., Gould M.N. Human metabolism of the experimental cancer therapeutic agent d-limonene. Cancer Chemother. Pharmacol., 35:31-37,1994 (a).
➢ Crowell P.L., Gould M.N. Chemoprevention and therapy of cancer by d-limonene. CRC. Crit. Rev. Oncogenesis., 5:1-22,1994 (b).
➢ Crowell P.L. et al. Dietary Phytochemicals in Cancer Prevention and Treatment. Plenum Press, New York, 1996,
➢ Crowell P.L. Prevention and therapy of cancer by dietary monoterpenes. J. Nutr., 129(3):775S-778S,1999.
➢ De Pooter H.L. et al. Essential oil of the leaves of three Pistacia species grown in Egypt. Flav. Fragr. J., 6:229-232,1991.
➢ Dietrich D.R. & Swenberg J.A. The presence of α2u-globulin is necessary for d-limonene promotion of male rat kidney tumors. Cancer Res., 51:3512-3517,1991.
➢ Elegbede J.A., Elson C.E., Qureshi A., Tanner M.A. & Gould M.N. Inhibition of DMBA-induced mammary cancer by the monoterpene d-limonene. Carcinogenesis., 5:661-665,1984.
➢ Elegbede J.A., Maltzman T.H., Verma A.K., Tanner M.A. & Gould M.N. Mouse skin tumor promoting activity of orange peel oil and d-limonene: a reevaluation. Carcinogenesis., 7:2047-2049,1986 (a). ➢
Elegbede J.A., Elson C.E., Tanner M.A., Qureshi A. &Gould M.N. Regression of rat primary mammary tumors following dietary d-limonene. J. Natl. Cancer Inst., 76:323-325,1986 (b).
➢ Elson C.E., Maltzman T.H., Boston J.L., Tanner M.A. & Gould M.N. Anti-carcinogenic activity of d-limonene during the initiation and promotion-progression stages of DMBA-induced rat mammary carcinogenesis. Carcinogenesis., 9:331-332,1988.
➢ Elson C.E., Yu S.G. The chemoprevention of cancer by mevalonate-derived constituents of fruits and vegetables. J Nutr., 124:607-614,1994.
➢ Elson C.E. Suppression of mevalonate pathway activities by dietary isoprenoids: protective roles in cancer roles in cancer and cardiovascuar disease. J.Nutr., 125:1666S-1672S,1995.
➢ Evans E., Arneson D., Kovatch R., Supko J., Morton T., Siemann L., Cannon R., Tomaszewski J., Smith A. Toxicology and pharmacology of perillyl alcohol (NSC-641066) in rats and dogs. Proc. Am. Assoc. Cancer Res., 36:366,1995.
➢ Farnsworth N.R et al. Medicinal plants in therapy. Bull. World Health Organ., 63:965-981,1985.
➢ Farnsworth N.R The role of ethnopharmacology in drug development. Ciba Found Symp., 154:2-11,1990.
➢ Giner-Larza Eva m. el al. On the anti-infiammatory and anti-phospholipase A2 activity of extract from lanostane-rich species. ETH.J., 73:61-69,2000.
➢ Gould M.N., Moore C.J., Zhang R., Wang B., Kennan W.S. & Haag J.D. Limonene chemoprevention of mammary carcinoma induction following direct in situ transfer of v-Ha-ras. Cancer Res., 54:3540-3543,1994.
➢ Haag J.D., Lindstrom M.J. & Gould M.N. Limonene-induced regression of mammary carcinomas. Cancer Res., 52:4021-4026,1992.
➢ Haag J.D. & Gould M.N. Mammary carcinoma regression induced by perillyl alcohol, a hydroxylated analog of limonene. Cancer. Chemother. Pharmacol., 34;477-483,1994.
➢ Hursting S.D., Perkins S.N., Haines D.C., Ward J.M.& Phang J.M. Chemoprevention of spontaneous tumorigenesis in p53-knockout mice. Cancer Res., 55:3949-3953, 1995.
➢ Huwez F.U. et al. Mastic Gum Kills Helicobacter pylori. N. Engl. J. Med., 339(26):365,1998.
➢ Jeffers L., Church D., Gould M. and Wilding G. The effect of perillyl alcohol on the proliferation of human prostatic cell lines. Proc. Am. Assoc. Cancer Res., 36:303, 1995.
➢ Jirtle R.L., Haag J:D., Ariazi E., Gould M.N. Increased mannose 6-phosphate/insulin-like growth factor II receptor and transforming growth fact b 1 levels during monoterpene-induced regression of mammary tumors. Cancer Res., 53:3849-3853, 1993.
➢ Kawamori T., Tanaka T., Hirose Y., Ohnishi M. & Mori H. Inhibitory effects of d-limonene on the development of colonic aberrant crypt foci induced by azoxymethane in F344 rats. Carcinogenesis., 17:369-372,1996.
➢ Kawata S., Nagase T., Yamasaki E., Ishiguro H., Matsuzwawa Y. Modulation of the mevalonate pathway and cell growth by pravastatin and d-limonene in a human hepatoma cell line (Hep G2). Br. J. Cancer., 69:1015-1020,1994.
➢ Magiatis P. et al. Chemical Composition and Antimicrobial Activity of the Essential Oils of Pistacia lentiscus var.chia. Planta Med., 65(8):749-752,1999.
> Maltzman T.H., Hurt L.M., Elson C.E., Tanner M.A. & Gould M.N. The prevention of nitrosomethylurea-induced mammary tumors by d-limonene and orange oil. Carcinogenesis., 10:781-785,1989.
➢ Marone P. et al. Bacterial activity of Pistacia lentiscus mastic gum against Helicobacter pylori. J. Chemoth., 13(6):611-614,2001.
➢ McNamee D. Limonene trial in cancer. Lancet., 342:801,1993.
➢ Mills J.J., Chari R.S., Boyer I.J., Gould M.N. & Jirtle R.L. Induction of apoptosis in liver tumors by the monoterpene perillyl alcohol. Cancer Res., 55:979-983,1995.
➢ Papageorgiou V.P. et al. GLC-MS computer analysis of the essential oil of mastic gum. Chim. Chronica, New Ser., 10:119-124,1981.
➢ Phillips L.R., Malspeis L. & Supko J.G. Pharmacokinetics of active drug metabolites after oral administration of perillyl alcohol, an investigational antineoplastic agent, to the dog. Drug Metab. Dispos., 23:676-680.
➢ Ray M., Kratz D., Lewis K., Hohl R.J. Effects of combinations of lovastatin and monoterpenes on ras processing. Proc of AACR., 36:428,1995.
➢ Ren Z. and Gould M.N. Inhibition of ubiquinone and cholesterol synthesis by the monoterpene perillyl alcohol. Cancer Lett.76:185-190, 1994.
➢ Rodbard D and Frazier GR. Statistical analysis of radioligand assay data. Methods Enzymol. 37:3-22;1975.
➢ Russin W.A., Hoesly J.D., Elson C.E., Tanner M.A. & Gould M.N. Inhibition of rat mammary carcinogenesis by monoterpenoids. Carcinogenesis., 10:2161-2165,1989.
➢ Sanz M.J. et al. In vivo hypotensive activity of Pistacia lentiscus L. Phytother. Res., 2(4):201-203,1988.
➢ Scurbis B., Markakis P. Essential oil of mastic gum. Int. Flavours Food Addit., 6:349,1975.
➢ Shoff S.M., Grummer M., Yatvin M.B. & Elson C.E. Concentration-dependent increase of murine P388 and B16 population doubling time by the acyclic monoterpene geraniol. Cancer Res., 51:37-42,1991.
➢ Stark M.J. et al. Chemotherapy of pancreatic cancer with the monoterpene perillyl alcohol. Cancer lett., 96(1):15-21,1995.
➢ Wattenberg L.W. Inhibition of neoplasia by minor dietary constituents. Cancer Res., 43:2448s-2453s,1983.
➢ Wattenberg L.W., Sparnins V.L. & Barany G. Inhibition of N-nitrosodiethylamine carcinogenesis in mice by naturally occurring organosulfur compounds and monoterpenes. Cancer Res., 49:2689-2694,1989.
➢ Wattenberg L.W., & Coccia J.B. Inhibition of 4-(methylnitrosoamino)-1-butanone carcinogenesis in mice by d-limonene and citrus fruit oils. Carcinogenesis., 12:115-117,1991.
➢ Wyllie S.G. et al. Volatile components of the fruits of Pistacia lentiscus. J. Food Sci., 55:1325-1326,1990.
➢ Yu S.G., Hildebrandt L.A. & Elson C.E. Geraniol, an inhibitors of mevalonate biosynthesis, suppresses the growth of hepatomas and melanomas transplanted to rats and mice. J. Nutr., 125:2763-2767,1995.

## Claims

1. Use of an essential oil obtained from a plant of Pistacia genus selected from p. Vera and p. Integerrima for manufacturing a medicament for treating or preventing cancer in a mammal, including a human, by administration of an effective amount.

2. The use of claim 1, wherein the plants ofPistacia genus selected from p. Vera and p. Integerrima are of European and Asiatic origin.

3. The use of claim I wherein the essential oil is an essential oil obtained from seeds of p. Vera of Italian origin.

4. The use of claim 3 wherein the essential oil is an essential oil obtained from seeds of p. Vera of Sicily.

5. The method of claim 1 wherein the essential oil is an essential oil from galls of p. Integerrima of Asiatic origin.

6. The method of claim 5 wherein the essential oil is an essential oil obtained from galls of p. Integerrima of Nepal.

7. The use of claim 1 wherein the cancer is breast adenocarcinoma and the essential oil is selected from the group consisting of essential oils obtained from nuts of p. Vera from Italy.

8. The use of claim 1 wherein the cancer is ovary adenocarcinoma and the essential oil is an essential oil obtained from galls of p. Integerrima from Nepal.

9. The use of claim 1 wherein the cancer is colon adenocarcinoma and the essential oil is are obtained from galls ofp. Integerrima from Nepal.

10. The use of (-)-bomyl acetate for manufacturing medicament for treating or preventing a cancer selected from the group consisting of colon adenocarcinoma and ovary adenocarcinoma.

## Patentansprüche

1. Verwendung eines etherischen Öls, das aus einer Pflanze der Gattung Pistacia, ausgewählt aus p. Vera und p. Integerrima, erhalten wird, zur Herstellung eines Medikaments zur Behandlung oder Prävention von Krebs bei einem Säuger, einschließlich eines Menschen, durch Verabreichung einer wirksamen Menge.

2. Verwendung gemäß Anspruch 1, wobei die Pflanzen der Gattung Pistacia, ausgewählt aus p. Vera und p. Integerrima, europäischer und asiatischer Herkunft sind.

3. Verwendung gemäß Anspruch 1, wobei das etherische Öl ein etherisches Öl ist, das aus Samen von p. Vera mit Herkunft aus Italien erhalten wird.

4. Verwendung gemäß Anspruch 3, wobei das etherische Öl ein etherisches Öl ist, das aus Samen von p. Vera aus Sizilien erhalten wird.

5. Verfahren gemäß Anspruch 1, wobei das etherische Öl ein etherisches Öl aus Gallen von p. Integerrima mit Herkunft aus Asien ist.

6. Verfahren gemäß Anspruch 5, wobei das etherische Öl ein etherisches Öl ist, das aus Gallen von p. Integerrima aus Nepal erhalten wird.

7. Verwendung gemäß Anspruch 1, wobei der Krebs Brustadenokarzinom ist und das etherische Öl ausgewählt ist aus der Gruppe, bestehend aus etherischen Ölen, die aus Nüssen von p. Vera aus Italien erhalten werden.

8. Verwendung gemäß Anspruch 1, wobei der Krebs Eierstockadenokarzinom ist und das etherische Öl ein etherisches Öl ist, das aus Gallen von p. Integerrima aus Nepal erhalten wird.

9. Verwendung gemäß Anspruch 1, wobei der Krebs Colonadenokarzinom ist und das etherische Öl aus Gallen von p. Integerrima aus Nepal erhalten wird.

10. Verwendung von (-)-Bornylacetat zur Herstellung eines Medikaments zur Behandlung oder Vorbeugung eines Krebses, ausgewählt aus der Gruppe, bestehend aus Colonadenokarzinom und Eierstockadenokaizinom.

## Revendications

1. Utilisation d'une huile essentielle obtenue à partir d'une plante du genre Pistacia choisie parmi *P*. *vera* et *P*. *integerrima* pour la fabrication d'un médicament pour traiter ou prévenir un cancer chez un mammifère, incluant un être humain, par administration d'une quantité efficace.

2. Utilisation selon la revendication 1, dans laquelle les plantes du genre Pistacia choisies parmi *P*. *vera* et *P*. *integerrima* sont d'origine européenne et asiatique.

3. Utilisation selon la revendication 1, dans laquelle l'huile essentielle est une huile essentielle obtenue à partir de graines de *P*. *vera* d'origine italienne.

4. Utilisation selon la revendication 3, dans laquelle l'huile essentielle est une huile essentielle obtenue à partir de graines de *P*. *vera* de Sicile.

5. Utilisation selon la revendication 1, dans laquelle l'huile essentielle est une huile essentielle obtenue à partir de galles de *P*. *integerrima* d'origine asiatique.

6. Utilisation selon la revendication 5, dans laquelle l'huile essentielle est une huile essentielle obtenue à partir de galles de *P*. *integerrima* du Népal.

7. Utilisation selon la revendication 1, dans laquelle le cancer est un adénocarcinome du sein et l'huile essentielle est choisie dans le groupe constitué par les huiles essentielles obtenues à partir de noix de *P*. *vera* d'Italie.

8. Utilisation selon la revendication 1, dans laquelle le cancer est un adénocarcinome de l'ovaire et l'huile essentielle est obtenue à partir de galles de *P*. *integerrima* du Népal.

9. Utilisation selon la revendication 1, dans laquelle le cancer est un adénocarcinome du côlon et l'huile essentielle est obtenue à partir de galles de *P*. *integerrima* du Népal.

10. Utilisation d'acétate de (-)-bornyle pour fabriquer un médicament pour traiter ou prévenir un cancer choisi dans le groupe constitué par un adénocarcinome du côlon et un adénocarcinome de l'ovaire.
